# EUROPEAN PATENT APPLICATION

(11) **EP 4 837 523 A1**
(43) Date of publication of application: **11.11.2026**
(21) Application number: 25736408.3
(22) Date of filing: 03.01.2025
(51) Int. Cl.: A61B 17/16, B23B 51/06

(54) **UNIDIRECTIONAL BONE BURR AND RONGEUR**

(30) Priority: 05.01.2024 CN 202420029047 U; 05.01.2024 CN 202420029042 U; 14.05.2024 CN 202421043861 U
(71) Applicant: Guizhou Zirui Technology Co., Ltd, Zunyi, Guizhou 563200 (CN)
(72) Inventor: LIU, Changfeng, Zunyi, Guizhou 563200 (CN); LI, Hongyuan, Zunyi, Guizhou 563200 (CN); LI, Huihua, Zunyi, Guizhou 563200 (CN); ZHANG, Jiashun, Zunyi, Guizhou 563200 (CN)
(74) Representative: Metida
(86) International application number: PCT/CN2025/070570
(87) International publication number: WO 2025/146170

(57) **Abstract**

The present disclosure provides a unidirectional bone burr and a rongeur. The unidirectional bone burr comprises a burr assembly comprising a burr head, a burr shank, and an outer sheath. The burr head is mounted at one end of the burr shank, and the outer sheath sleeves the burr shank. The burr shank is rotatable relative to the outer sheath to drive rotation of the burr head. The burr assembly is detachably connected to a power source assembly, with cooling channels thereof communicating with each other, so as to simultaneously cool a micro motor, a burr shaft, and the burr head through a single water injection, thereby achieving a good cooling effect. The burr shaft with the split design, by means of a fail-safe device, ensures that the burr shank and the burr head do not disengage from the outer sheath after failure of the burr shank and a metal wire, thereby improving surgical safety. A waterproof assembly secures the burr shaft with the split design by means of the fail-safe device, thereby ensuring that the burr shank and the burr head do not disengage from the outer sheath after failure of the burr shaft and the metal wire, thereby improving surgical safety. The rongeur strengthens the structural rigidity of a guide groove by increasing the wall thickness of the guide groove.

## Description

### TECHNICAL FIELD

The present application relates to the technical field of bone burr, and specifically to a unidirectional bone burr and a rongeur.

### BACKGROUND

In some orthopaedic operations, it is sometimes necessary to grind a bone tissue in the body of a patient, so an orthopaedic bone burr is developed. When the bone tissue grinding operation is performed in an operation, the conventional manual bone burr has low efficiency, which is not convenient for observing the operation position, and the force is not easy to control, such that the bone tissue with bad toughness around the operation position is not easy to be cut, and also likely to cause unnecessary damage to the soft tissue with good toughness around, especially to the soft tissue around the bone tissue.

In the Chinese patent application document published as CN111973249A, a water-cooling bone burr for operation is disclosed, cooling water flows into a supporting rod assembly through a cooling device, and flows out of the cooling bone burr along a burr shank, a driving device and the bone burr are integrally designed in the technical solution. It is not capable of speeding up the output of the driving device, and there is no fail-safe for the bone burr.

In the Chinese patent application document published as CN215018430U, an eccentric sheath bone burr is disclosed, by disposing a bent bone burr and a sheath with an eccentric hole, further disposing a cooling device capable of flowing out of the cooling bone burr along a burr shank. The technical solution is also an integral design, and it is not capable of speeding up the output of the driving device, and there is no fail-safe for the bone burr.

In the Chinese patent application document published as CN216455184U, a bone burr for operation is disclosed. In the technical solution, a speed changing device is connected to an output end of a motor such that the rotation of the bone burr is speeded up, meanwhile, the rotation of a speed-increasing impeller drives the cooling water to quickly flow, such that the aim of fast washing and cooling the bone burr is realized. The bone burr of the technical solution rotates to drive the impeller to rotate, the cooling water flows through the impeller, so there is a sealing problem, and the water-cooling solution of the technical solution is not capable of cooling the motor, and there is no fail-safe for the bone burr.

In the Chinese patent application document published as CN216535426U, a cooling bone burr for operation is disclosed. The rotation of an impeller is driven by a burr shank, a centrifugal force generated by the rotation of the impeller drives cooling water to quickly flow. The bone burr of the technical solution rotates to drive the impeller to rotate, the cooling water flows through the impeller, so there is a sealing problem, and the water-cooling solution of the technical solution is not capable of cooling the motor, and there is no fail-safe for the bone burr.

In the Chinese patent application document published as CN218606745U, a water outlet bone burr with an endoscopic supporting rod is disclosed. The bone burr is driven to rotate by a transmission device in a shank, realizing a quick rotation of an output end, that is, the bone burr is driven to quickly rotate by a low speed motor. The cooling water in an orthopaedic operation can only be discharged through a cutter channel, and a water sheath is independently disposed. The motor is not capable of being cooled, and a rod piece driving the bone burr to rotate is not capable of being cooled, the cooling effect is not good, and there is no fail-safe for the bone burr.

In the Chinese patent application document published as CN116531043A, a bone burr for operation is disclosed. A shank assembly is provided with a cooling water channel connected to a cavity groove, the cooling water channel and the cavity groove introduce cooling water to the bone burr, a vibration generated by the bone burr in a rotating process is dampened by an external elastic sheet via a propping action so as to meet the purpose of stable grinding. The cooling solution of the technical solution is not capable of cooling the motor, the sealing of the cooling flow passage is bad, and there is no fail-safe for the bone burr.

In the Chinese patent application document published as CN219700036U, a bone burr for operation is disclosed. Cooling water is introduced to the bone burr by a cooling water channel and a cavity groove. The cooling solution of the technical solution is not capable of cooling a motor, the sealing of the cooling water channel is bad, and there is no fail-safe for the bone burr.

At present, the market urgently needs a bone burr device capable of cooling a driving device, a transmission device and a bone burr of the bone burr device at the same time, as well as a cooling flow passage has good sealing, and has fail-safe for the bone burr.

### SUMMARY

To overcome the defects in the prior art, the present disclosure aims to provide a unidirectional bone burr and a rongeur.

The unidirectional bone burr according to the present disclosure, comprises a burr assembly, the burr assembly comprises a burr head, a burr shank and an outer sheath, the burr head is mounted at one end of the burr shank, the outer sheath sleeves the burr shank;
the burr shank is rotatable relative to the outer sheath to drive rotation of the burr head.

Preferably, a power source assembly and a cooling system is further comprised, the burr assembly is detachably connected to the power source assembly; an output end of the power source assembly outputs torque; the burr assembly comprises the burr head and a driving assembly, the driving assembly connects the burr head and the output end of the power source assembly in transmission; the cooling system cools the power source assembly and the burr assembly.

Preferably, the cooling system is integrated inside the burr assembly and the power source assembly, a water inlet of the cooling system is disposed at one end of the power source assembly far away from the burr assembly, a water outlet of the power source assembly is connected to a water inlet of the burr assembly, a water outlet of the burr assembly is close to the burr head and is capable of cooling the burr head.

Preferably, the power source assembly comprises a micro-motor, a motor shell and a cooling sheath, the cooling sheath sleeves an outer part of the micro-motor, the motor shell sleeves an outer part of the cooling sheath, a cooling channel allowing the cooling liquid to pass through is formed between the cooling sheath and the motor shell, a water inlet of the cooling channel is disposed at one end of the motor shell far away from the burr assembly, a water outlet of the cooling channel is disposed at one end of the motor shell close to the burr assembly;
a shell of the burr assembly is detachably connected to the motor shell, the detachable connection mode of the shell of the burr assembly and the motor shell comprises one or more selected from the following known connecting structures or components with detachable function: a screw thread connection, a clamping connection, a male-female connection, an elastic block, and a clamp, etc., but is not limited thereto. An output end of the micro-motor is provided with a connecting female header, a transmission shaft of the driving assembly is provided with a connecting male header, the connecting female header and the connecting male header are detachably connected.

Preferably, the driving assembly is disposed inside the shell of the burr assembly, the driving assembly comprises a transmission shaft and a gear speed changing device, a power input end of the gear speed changing device is connected to the transmission shaft in transmission, a power output end of the gear speed changing device is in transmission connection with the burr head through a burr shaft.

Preferably, the burr shaft is of an integral structure or a split structure, the burr shaft extends outside of the shell of the burr assembly, and there is a gap between the burr shaft and the shell of the burr assembly to form the water outlet of the burr assembly;
the burr shaft of the integral structure connects the burr head to an output end of the gear speed changing device in transmission;
the burr shaft of the split structure comprises a metal wire and the burr shank, one end of the metal wire is in transmission connection with the gear speed changing device, one end of the burr shank is connected to the burr head, and the other end of the metal wire is in transmission connection with the other end of the burr head through a connecting assembly.

Preferably, the connecting assembly comprises a transmission connecting sleeve, one end of the burr shank far away from the burr head is provided with a deep connecting hole, one end of the transmission connecting sleeve extends into the deep connecting hole and is fixedly connected to an inner wall of the deep connecting hole, one end of the transmission connecting sleeve located outside the deep connection hole is a fail-safe sleeve, an end surface of the fail-safe sleeve is fixedly connected to the end surface of the burr head;
the metal wire extends into the transmission connecting sleeve and extends an end part outside, the metal wire is fixedly connected to an inner wall of the transmission connecting sleeve.

Preferably, an outer part of the burr shank is sleeved with an elastic sheet sheath, an outer part of the elastic sheet sheath is sleeved with a water passing sleeve, a supporting sleeve is disposed between the water passing sleeve and the shell of the burr assembly.

Preferably, the connecting assembly comprises a transmission connecting sheath, the transmission connecting sheath is sleeved with a burr shank and a metal wire, the transmission connecting sheath is respectively fixedly connected to both the burr shank and the metal wire, the metal wire is sleeved with a fail-safe sheath, the transmission connecting sheath extends into the fail-safe sheath, an outer wall of the transmission connecting sheath is fixedly connected to a fail-safe block, the fail-safe block is embedded in an inner wall of the fail-safe sheath.

Preferably, a waterproof assembly is disposed between the cooling system integrated inside the burr assembly and the gear speed changing device.

A medical bone burr according to the present disclosure, comprises: a bone burr assembly for grinding bone tissue, the bone burr assembly comprises a burr head and a bendable burr shank, the burr is mounted at one end of the burr shank; and
a supporting assembly comprising an outer sheath with a water outlet hole on the side face, the outer sheath sleeves the burr shank, a gap between the outer sheath and the burr shank forms a water injection channel, the water outlet hole is connected to the water injection channel.

Preferably, a driving assembly is further comprised, the driving assembly comprises a transmission shaft and a transmission part, a power input end of the transmission part is connected to the transmission shaft, a power output end of the transmission part is connected to the burr shank;
the transmission part comprises a driving gear and a driven gear engaged with each other, the driving gear is mounted on the transmission shaft, the driven gear is mounted at one end of the burr shank far away from the burr head, a bearing is mounted between the outer sheath and the burr shank, the bearing is located at one end of the outer sheath close to the driven gear.

Preferably, the supporting assembly further comprises an elastic sheet sheath, the elastic sheet sheath sleeves the burr shank and is close to the burr head, the elastic sheet sheath is provided with a damping elastic sheet;
the outer sheath is bent, one end of the outer sheath far away from the burr head is provided with a water supply groove, the water supply groove is connected to the water injection channel;
a water passing sheath is further mounted between the outer sheath and the burr shank, the water passing sheath sleeves the elastic sheet sheath, the water passing sheath comprises a big sheath and a small sheath which are fixedly connected, the big sheath is close to the burr head, and an outer wall of the big sheath inserted into an outer sheath part is adhered with the inner wall of the outer sheath, the small sheath is sleeved with a first arc-shaped sleeve, an arc outer wall of the first arc-shaped sleeve is adhered with the inner wall of the outer sheath, an axial gap formed by the first arc-shaped sleeve sleeved on the small sheath is connected to the water outlet hole;
a supporting sleeve is further mounted between the outer sheath and the burr shank, the supporting sleeve comprises a sleeve and a second arc-shaped sleeve sleeved on the sleeve, the sleeve sleeves the burr shank, an arc-shaped outer wall of the second arc-shaped sleeve is adhered to the inner wall of the outer sheath, a gap formed between the second arc-shaped sleeve and the outer sheath forms a part of the water injection channel;
a supporting sheath is further mounted between the outer sheath and the burr shank, the supporting sleeve is located between the water passing sheath and the supporting sheath, one end of the supporting sheath is provided with a flat square, the water outlet of the water passing groove is located on the flat square.

Preferably, a front shell, a spiral water-cooling interface and a sealing sleeve are further comprised, the front shell and the sealing sleeve are hermetically connected, one end of the outer sheath and one end of the burr shank, far away from the burr head, are inserted into the front shell, the water supply groove is located in the front shell, an outer wall of the spiral water-cooling interface is provided with a water flow channel and an axial through groove, the front shell is provided with a water supply hole, the water supply hole is located in the sealing sleeve, the water supply hole is connected to the water supply groove, a water inlet end of the axial through groove is connected to the water flow channel, a water outlet end of the axial through groove is connected to the water supply hole;
a water-cooling sleeve is further disposed between the spiral water-cooling interface and the sealing sleeve, the water-cooling sleeve sleeves one end of the spiral water-cooling interface far away from the front shell, the water-cooling sleeve is provided with an axial water groove, the axial water groove is connected to a water inlet end of a spiral channel.

An orthopaedic bone burr according to the present disclosure, comprising:
a burr assembly for grinding bone tissue, the burr assembly comprises a burr head and a burr shank, the burr head is mounted at one end of the burr shank, the burr shank has elasticity and can be bent;
a driving assembly for driving the burr assembly to rotate;
a supporting assembly, comprising an outer sheath and an arc-shaped guide sleeve, the outer sheath sleeves the burr shank, the arc-shaped guide sleeve is located between the outer sheath and the burr shank;
a sheath sleeve for hiding and wrapping a part of the burr, the sheath sleeve is connected to the outer sheath, an axial line of the sheath sleeve is crossed with an axial line of the outer sheath.

Preferably, the supporting assembly further comprises a first sleeve and a first supporting sheath, the first sleeve sleeves the burr shank, and the first sleeve is close to the burr head, the first supporting sheath sleeves the first sleeve, and the first sleeve is in clearance fit with the first supporting sheath;
the sheath sleeve is provided with an abdication groove and an eccentric hole, the abdication groove is connected to the eccentric hole, the burr head and the first sleeve and one end of the first supporting sheath are located in the abdication groove;
the burr shank comprises a connection shank and a long shank, one end of the connection shank is connected to the burr head, the other end is connected to the long shank, the long shank comprises an elastic bending section and a straight section, the elastic bending section is composed of multiple short shanks;
a second sleeve is further disposed between the outer sheath and the burr shank, the second sleeve can be bent, and the second sleeve sleeves the burr shank;
the arc-shaped guide sleeve sleeves the second sleeve, an arc-shaped outer wall of the arc-shaped guide sleeve is adhered to an inner wall of the outer sheath; and
an internal path of the arc-shaped guide sleeve first ascends and then descends from one end far away from the burr head to one end close to the burr head; the second sleeve conforms to an arc-shaped contour inside the arc-shaped guide sleeve.

Preferably, a second supporting sheath is further disposed between the outer sheath and the burr shank, the second supporting sheath sleeves the burr shank, the second sleeve is located between the first sleeve and the second supporting sheath; and
an outer wall of the second supporting sheath is provided with a flat square, a gap between the flat square and the outer sheath, and a gap between the arc-shaped guide sleeve and the outer sheath form a water injection passage through which the cooling water flows.

Preferably, the driving assembly comprises an input shaft, one end of the burr shank far away from the burr head is connected to the input shaft; the driving assembly further comprises a bearing sleeve, one end of the bearing sleeve is mounted with a bearing, the bearing sleeve is disposed on a power output end of the input shaft; and
a front shell, a water-cooling sleeve and a sealing sleeve are further comprised, a stepped hole is disposed inside the front shell, one end of the outer sheath, one end of the burr shank and one end of the second supporting sheath ,far away from the burr head, are inserted into the front shell from a small hole of the stepped hole, the front shell is hermetically connected to the sealing sleeve, the sealing sleeve sleeves the water-cooling sleeve, one end of the water-cooling sleeve sleeves the front shell, an outer wall of the water-cooling sleeve is provided with a spiral channel, the front shell is provided with a water supply hole, a water inlet end of the water supply hole is connected to a water outlet end of the spiral channel, a water outlet end of the water supply hole is connected to the water injection channel, an outer wall of one end of the bearing sleeve is propped against an inner wall of the front shell to form sealing.

A single water injection burr mechanism according to the present disclosure, comprises a burr assembly and an outer sheath, the burr assembly comprises a burr head and a burr shank connected to the burr head, a first cavity is provided inside the burr head, the burr head is provided with a water outlet hole, the water outlet hole is connected to the first cavity, one end of the burr shank close to the burr head is provided with a second cavity, the second cavity is connected to the first cavity; and
the outer sheath sleeves the burr shank, the outer sheath is provided with a first water inlet hole, the first water inlet hole is used for injecting water to the inner part of the outer sheath, a water flow channel is formed between an inner wall of the outer sheath and an outer wall of the burr shank, the water flow channel is connected to both the first water inlet hole and the second cavity.

Preferably, a supporting guide sheath is further comprised, the supporting guide sheath sleeves the burr shank, and the inner wall of the outer sheath is contacted with an outer wall of the supporting guide sheath, an outer wall of the supporting guide sheath is provided with a first flat square, a gap between the first flat square and the inner wall of the outer sheath forms a first channel, the first channel is a part of the water flow channel, the first water inlet hole is located at one end of the first flat square, and the first water inlet hole is connected to the first channel;
a supporting cavity sheath is sleeved between the outer sheath and the burr shank, a second channel is disposed inside the supporting cavity sheath, the second channel is connected to the first channel, the second channel is also a part of the water flow channel;
a stepped hole and a guide groove are provided inside the supporting cavity sheath, the stepped hole is located at one end of the supporting cavity sheath far away from the burr head, the guide groove is opened on an inner wall of the supporting cavity sheath, the guide groove is connected to the stepped hole to form a second channel, the guide groove is connected to the second cavity;
the burr shank is provided with a second water inlet hole on a wall of the second cavity, the second water inlet hole is connected to both the guide groove and the second cavity; and
a front supporting sheath and a back supporting sheath are further disposed between the supporting cavity sheath and the burr shank, the front supporting sheath sleeves one end of the burr shank close to the burr head, one end of the front supporting sheath is close to the burr head, the other end is inserted into the supporting cavity sheath and keeps away from the second water inlet hole, the back supporting sheath also sleeves the burr shank and keeps away from the second water inlet hole; a position of the back supporting sheath is corresponding to a position of the guide groove.

A bone rongeur according to the present disclosure, characterized by comprising a burr assembly, the burr assembly comprises a burr head, a burr shank and an outer sheath, the burr head is mounted at one end of the burr shank, and the outer sheath sleeves the burr shank, the burr shank is rotatable relative to the outer sheath to drive rotation of the burr head; and
further comprising a sheath sleeve, an adjusting mechanism and a shank, the adjusting mechanism and the burr assembly are configured on the shank, the adjusting mechanism is provided with a pressing shank, the adjusting mechanism controls the sheath sleeve to move back and forth relative to the burr head.

Preferably, the pressing shank is fixedly connected to a guide rod; the guide rod sleeves the outer sheath; the guide rod connects the sheath sleeve to the pressing shank; a reset spring is disposed between the pressing shank and the shank; and
the shank, the guide rod and the sheath sleeve as a whole can move toward or away from the shank along a direction of the outer sheath.

Preferably, a shape of the inner wall of an end part of the sheath sleeve is matched with a shape of the end surface of the burr head, and an edge of the end part of the sheath sleeve is provided with a tip, a peripheral side wall of the sheath sleeve is provided with a guide groove, a side edge of the burr head enters into the guide groove; and
a gasket is disposed between the sheath sleeve and the outer sheath such that a central axial line of the burr head is not overlapped with that of the sheath sleeve.

Preferably, a front end of the bone burr is provided with a cutting edge, while its circumferential surface is smooth.

Compared with the prior art, one or more embodiments of the present disclosure have the following beneficial effects:
1. The burr assembly and the power source assembly are detachably connected, and the cooling channels of the two are connected. The micro-motor, the burr shaft and the burr head can be cooled at the same time by injecting water once, and the cooling effect is good.
2. With the detachable designed burr shaft and the fail-safe device, it is ensured that the burr shank and the burr head will not fall from the outer sheath after the failure of the burr shank and the metal wire, thereby improving the safety of the operation.
3. Through the cooperation of the first waterproof sheath, the second waterproof sheath, the structure supporting sheath and the lining sheath, the gear speed changing device is isolated from the cooling system, such that the cooling liquid will not enter into the gear speed changing device, thereby improving the service life of the device.
4. By adjusting the gear ratio of the driving gear and the driven gear to realize variable-speed rotation, the cost of the motor is saved and meanwhile the operation time is shortened, and the wound surface of the bone tissue grinding position is regular and stable, thereby improving the processing effect of bone tissue grinding or repairing. On the other hand, the disposition of the elastic sheet sheath and the design of the outer sheath in the bending state can suppress the vibration generated in the rotation process of the burr head to a certain extent, so as to meet the purpose of stable grinding. Moreover, through the external water injection mechanism, the cooling water is injected into the axial water groove on the water-cooling sleeve, the cooling water flows into the spiral channel through the axial water groove, then flows into the water supply hole of the front shell through the axial through groove, enters into the outer sheath through the water supply hole and the water supply groove, finally flows out from the water outlet hole of the outer sheath, it is used for cooling and washing the grinding position, thereby improving the comfort of the patient and improving the grinding efficiency.
5. By adopting the burr shank with a partial cavity and the burr head with a cavity, the injected cooling water flows out from the water outlet hole on the burr head, which effectively reaches the burr head and grinding position, thereby improving cooling efficiency and cooling effect. The structure inside the bone burr is simple and it is convenient to use.
6. With the elastic bending section of the burr shank, in the rotating process, when the burr head contacts with the bone tissue and the grinding position is not confirmed, there is an abdication flick under the action of the elastic force; after the grinding position is confirmed, the burr head is controlled to extrude the grinding position, the elastic bending section is bent, which can ensure the accuracy of the grinding position to a certain extent, meanwhile, it can guide and provide a better operation angle. The disposition of the sheath sleeve can shield a part of the grinding position of the burr head, thereby effectively protecting the soft tissue at the non-operation position, and the disposition of the abdication groove can avoid the rotation of the burr head and interfere with the sheath sleeve, so as to adapt to the burr head with a larger size. The larger the area of the burr head protruding from the sheath sleeve on one side, the easier it is to adjust the angle of the grinding operation, thereby improving the working efficiency.
7. By disposing the central axis of the bone rongeur burr not overlapped with that of the sheath sleeve, the groove wall of the guide groove is thicker, and the structural strength of the guide groove is increased.

### BRIEF DESCRIPTION OF THE DRAWINGS

Other features, objects and advantages of the present disclosure will be more apparent from the following detailed description of non-limiting embodiments with reference to the accompanying drawings.
FIG. 1 is a sectional view of an overall structure of a burr assembly according to a first embodiment of the present disclosure;
FIG. 2 is a sectional view of an overall structure of a power source assembly according to a first embodiment of the present disclosure;
FIG. 3 is a sectional view of an assembly structure of a waterproof assembly according to a first embodiment of the present disclosure;
FIG. 4 is a schematic view of an connection structure of a burr shank and a steel wire hose according to a first embodiment of the present disclosure;
FIG. 5 is an exploded view of an connection structure between a burr shank and a steel wire hose according to a first embodiment of the present disclosure;
FIG. 6 is a schematic view of another connection structure between a burr shank and a steel wire hose according to a first embodiment of the present disclosure;
FIG. 7 is a schematic diagram of an explosion structure of a bone burr according to a second embodiment of the present disclosure;
FIG. 8 is a schematic view of a structure of a bone burr according to a second embodiment of the present disclosure;
FIG. 9 is a sectional view of a bone burr according to a second embodiment of the present disclosure;
FIG. 10 is an enlarged view of a position A in FIG. 9 in a second embodiment of the present disclosure;
FIG. 11 is an enlarged view of a position B in FIG. 9 in a second embodiment of the present disclosure;
FIG. 12 is an enlarged structrual schematic view of an outer sheath according to a second embodiment of the present disclosure;
FIG. 13 is an enlarged structrual schematic view of a water passing sheath according to a second embodiment of the present disclosure;
FIG. 14 is an enlarged structrual schematic view of a supporting sheath according to a second embodiment of the present disclosure;
FIG. 15 is an exploded structrual schematic view of a bone burr according to a third embodiment of the present disclosure;
FIG. 16 is a structrual schematic view of a bone burr according to a third embodiment of the present disclosure;
FIG. 17 is a sectional view of a bone burr according to a third embodiment of the present disclosure;
FIG. 18 is an enlarged structural schematic view of a position A in FIG. 17 according to a third embodiment of the present disclosure;
FIG. 19 is an enlarged structural schematic view of a position B in FIG. 17 according to a third embodiment of the present disclosure;
FIG. 20 is an enlarged structural schematic view of a burr assembly according to a third embodiment of the present disclosure;
FIG. 21 is an enlarged structural schematic view of an arc-shaped guide sleeve according to a third embodiment of the present disclosure;
FIG. 22 is an enlarged structural schematic view of a second supporting sheath according to a third embodiment of the present disclosure;
FIG. 23 is a sectional view of a burr mechanism according to a fourth embodiment of the present disclosure;
FIG. 24 is a structural schematic view of a burr mechanism according to a fourth embodiment of the present disclosure;
FIG. 25 is a partial enlarged structural schematic view of a bone burr assembly according to a fourth embodiment of the present disclosure;
FIG. 26 is an enlarged structural schematic view of a supporting cavity sheath according to a fourth embodiment of the present disclosure;
FIG. 27 is an exploded structural schematic view of a bone burr according to a fourth embodiment of the present disclosure;
FIG. 28 is a sectional view of a bone burr according to a fourth embodiment of the present disclosure;
FIG. 29 is a structural schematic view of a bone burr according to a fourth embodiment of the present disclosure;
FIG. 30 is a schematic view of an overall structure of a rongeur according to a fifth embodiment of the present disclosure.

It is shown in the figure as follows: 110, burr head; 120, burr shank; 210, transmission shaft; 220, driving gear; 230, driven gear; 240, bearing; 1001, micro motor; 1002, motor shell; 1003 cooling sheath; 1004, connecting female header; 1005, transmission shaft; 1006, connecting male header; 1007, front shell; 1008, spiral water-cooling sleeve; 1009, water-cooling sleeve; 1010, sealing sleeve; 1011, outer sheath; 1012, burr shaft; 1013, metal wire; 1014, hard connecting sheath; 1015, structure supporting sheath; 1016, water supply groove; 1017, first waterproof sheath; 1018, second waterproof sheath; 1019, lining sheath; 1020, structure retaining sheath; 1021, transmission connecting sleeve; 1022, deep connecting hole; 1023, fail-safe sleeve; 1024, elastic sheet sheath; 1025, water passing sleeve; 1026, supporting sleeve; 1027, transmission connecting sheath; 1028, fail-safe sheath; 1029, fail-safe block; 1030, external supporting sleeve; 1031, water injection flow passage sheath; 1032, dredging hole; 1033, cooling water interface; 1034, oil injection hole; 2310, outer sheath; 2311, water outlet hole; 2312, water supply groove; 2320, elastic sheet sheath; 2330, water passing sheath; 2340, first arc-shaped sleeve; 2350, supporting sleeve; 2351, sleeve; 2352, second arc-shaped sleeve; 2360, supporting sheath; 2361, flat square; 2410, front shell; 2411, clamping groove; 2412, water supply hole; 2420, spiral water-cooling interface; 2421, axial through groove; 2422, water flow channel; 2430, sealing sleeve; 2431, snap ring; 2440, water-cooling sleeve; 2441, axial water groove; 3000, cavity; 3121, connecting shank; 3122, long shank; 31221, elastic bending section; 31222, straight section; 3210, input shaft; 3220, bearing sleeve; 3230, bearing; 3310, outer sheath; 3320, first sleeve; 3330, first supporting sheath; 3340, second sleeve; 3350, arc-shaped guide sleeve; 3360, second supporting sheath; 3361, flat square; 3400 sheath sleeve; 3410, abdication groove; 3510, front shell; 3511, clamping groove; 3512, water supply hole; 3520, water-cooling sleeve; 3521, notch; 3530, sealing sleeve; 3531, snap ring; 3540, water-cooling interface; 4100, burr assembly; 4111, first cavity; 4112, water outlet hole; 4121, second cavity; 41201, first section; 41202, second section; 4122, second water inlet hole; 4200, outer sheath; 4210, first water inlet hole; 4220, through hole; 4230, water inlet groove; 4300, supporting guide sheath; 4310, first flat square; 4320 blind hole; 4400, supporting cavity sheath; 4410, stepped hole; 4420, guide groove; 4510, front supporting sheath; 4520, back supporting sheath; 4610, shell; 4611, first shell; 46111, ring groove; 46112, third water inlet hole; 4612, second shell; 46121, retaining ring; 4620, input shaft; 4700, water-cooling interface; 4710, first water injection groove; 4720 spiral channel; 4730, second water injection groove; 4810, driven gear; 4820, driving gear; 4830, bearing; 4001, first channel; 4002, second channel; 5001, outer sheath; 5002, sheath sleeve; 5003, shank; 5004, guide rod; 5005, reset spring; 5006, guide groove; 5007, gasket.

### DETAILED DESCRIPTIONS OF THE EMBODIMENTS

The present disclosure will be described in detail below with reference to the specific embodiments. The following examples will help those skilled in the art to further understand the present disclosure, but are not intended to limit the present disclosure in any form. It should be noted that various changes and modifications could be made to those of ordinary skill in the art without departing from the spirit of the present disclosure. All these belong to the protection scope of the present disclosure.

### Embodiment 1

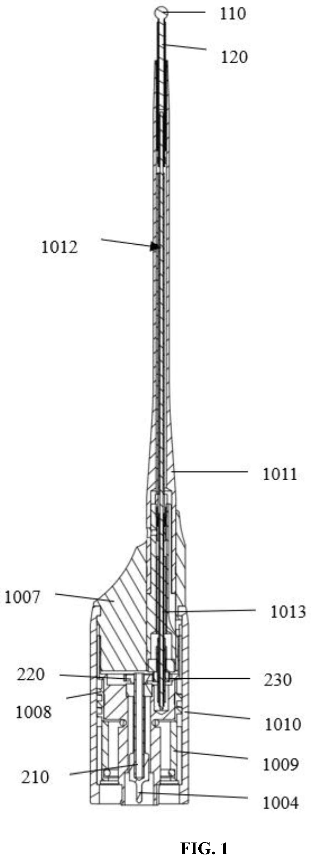

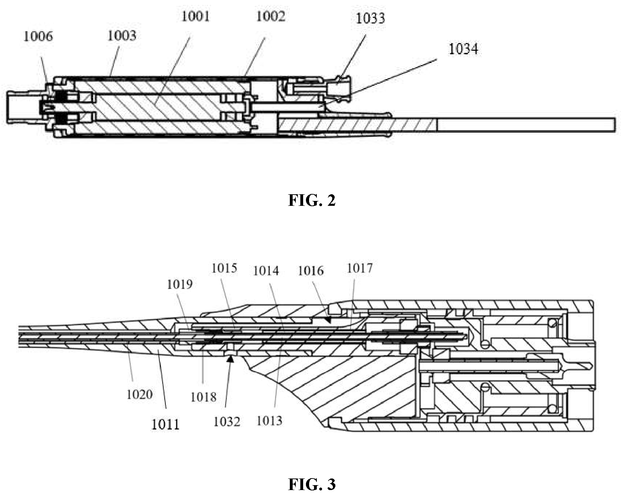

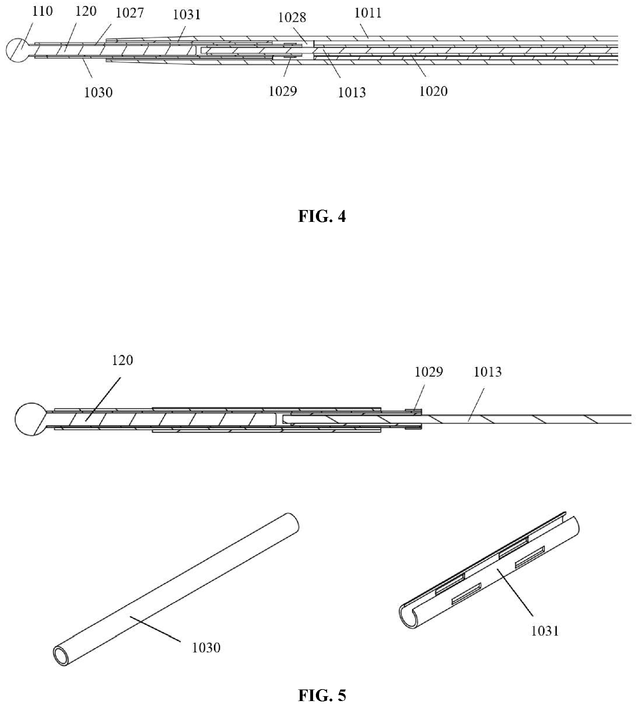

As shown in FIGS. 1 to 6, a unidirectional bone burr according to the present disclosure, comprises a burr assembly, a power source assembly, and a cooling system. The burr head 110 is detachably connected to the power source assembly. The output end of the power source assembly outputs torque. The burr head 110 assembly includes a burr head 110 and a drive assembly that connects the burr head 110 to the output of the power source assembly in transmission. The cooling system cools the power source assembly and the burr head 110 assembly.

The cooling system is integrated inside the burr head 110 assembly and the power source assembly, the water inlet of the cooling system is disposed at one end of the power source assembly far away from the burr head 110 assembly, the water outlet of the power source assembly is connected to the water inlet of the burr head 110 assembly, The water outlet of the burr head 110 assembly is proximate to the burr head 110 and is capable of cooling the burr head 110.

The power source assembly comprises a micro-motor 1001, a motor shell 1002 and a cooling sheath 1003, the cooling sheath 1003 sleeves the outer part of the micro-motor 1001, the motor shell 1002 sleeves the outer part of the cooling sheath 1003, a cooling channel allowing the cooling liquid to pass through is formed between the cooling sheath 1003 and the motor shell 1002, a cooling system in the power source assembly is formed, the water inlet of the cooling channel is disposed at one end of the motor shell 1002 far away from the burr head 110 assembly, a cooling water interface 1033 is formed at the water inlet of the cooling channel, and the cooling water interface 1033 can be externally connected to the infusion sheath of the hospital. The water outlet of the cooling channel is disposed at one end of the motor shell 1002 close to the burr head 110 assembly. It should be noted that the technical solution of the present disclosure does not specifically define the shape of the cooling liquid passage on the cooling sheath 1003. In a preferred embodiment, the cooling liquid passage on the cooling sheath 1003 may be a helical channel.

Further, the motor shell 1002 is provided with an oil injection hole 1034 through which the micro-motor 1001 can be fueled during actual use. Specifically, the position of the oil injection hole 1034 on the motor shell may be the end of the motor shell 1002 away from the burr assembly, and may also be on the peripheral side wall of the motor shell 1002.

The shell of the burr assembly is detachably connected to the motor shell 1002, the detachable connection mode of the shell of the burr assembly and the motor shell 1002 comprises one or more selected from the following known connecting structures or components with detachable function of: a screw thread connection, a clamping connection, a male-female connection, an elastic block, and a clamp, etc., but is not limited thereto. The output end of the micro-motor 1001 is provided with a connecting female header 1004, the transmission shaft 210 of the driving assembly is provided with a connecting male header 1006, the connecting female header 1004 and the connecting male header 1006 are detachably connected.

The burr head 110 assembly comprises a front shell 1007, a spiral water-cooling sleeve 1008, a water-cooling sleeve 1009, a sealing sleeve 1010 and an outer sheath 1011, the front shell 1007 and the sealing sleeve 1010 are hermetically connected, specifically, the outer wall of the front shell 1007 is provided with an annular clamping groove, the sealing sleeve 1010 is provided with a clamping ring, the clamping ring is embedded in the clamping groove, and sealed by glue. One end of the outer sheath 1011 far away from the burr head 110 is inserted into the front shell 1007, the spiral water-cooling sleeve 1008 and the water-cooling sleeve 1009 are located in the sealing sleeve 1010, the outer wall of the spiral water-cooling sleeve 1008 is provided with a spiral channel and an axial through groove, the front shell 1007 is provided with a water supply hole, the water supply hole is located in the sealing sleeve 1010, the water inlet end of the axial through groove is connected to the spiral channel, the water outlet end of the axial through groove is connected to the water supply hole. The water-cooling sleeve 1009 sleeves one end of the spiral water-cooling sleeve 1008 far away from the front shell 1007, the water-cooling sleeve 1009 is provided with an axial water groove, the axial water groove is connected to the water inlet end of the spiral channel. The outer sheath 1011 is provided with a water supply groove 1016, the water supply groove 1016 connects the water supply hole with the inner cavity of the outer sheath 1011. The inner wall of the front shell 1007 and the water supply groove 1016 of the outer sheath 1011 and the inner wall of the outer sheath 1011 and the flat square on the supporting sheath form a cavity through which the cooling water flows. The water inlet of the axial water groove of the water-cooling sleeve 1009 is located on the end surface of the burr head 110 assembly close to the power source assembly, the water inlet of the axial water groove of the water-cooling sleeve 1009 can be connected to the water outlet of the cooling channel power source assembly. Preferably, a sealing ring is provided at the water inlet of the axial water groove of the water-cooling sleeve 1009, and the sealing ring seals the connection between the water inlet of the axial water groove of the water-cooling sleeve 1009 and the water outlet of the cooling channel on the motor shell 1002 to prevent the cooling liquid from entering the inner cavity of the water-cooling sleeve 1009. Further, a sealing ring is also provided at the joint of the water-cooling sleeve 1009 and the spiral water-cooling sleeve 1008 to prevent the cooling liquid from entering the burr assembly from the joint of the water-cooling sleeve 1009 and the spiral water-cooling sleeve 1008, thereby improving the water-proof protection of the internal device of the burr assembly.

The driving assembly is disposed in the shell of the burr head 110 assembly, the driving assembly comprises a transmission shaft 210 and a gear speed changing device, the power input end of the gear speed changing device is in transmission connection with the transmission shaft 210, the power output end of the gear speed changing device is in transmission connection with the burr head 110 through the burr shaft 1012. The gear speed changing device is mounted in the inner cavity of the spiral water-cooling sleeve 1008, one end of the burr shaft 1012 is in transmission connection with the output end of the gear speed changing device, The burr shaft 1012 passes through the inner cavity of the outer sheath 1011 and extends from one end of the outer sheath 1011 away from the gear speed changing device to be in transmission connection with the burr head 110, and the inner cavity of the outer sheath 1011 is integrated with a cooling flow passage, the cooling liquid enters into the cooling flow passage in the inner cavity of the outer sheath 1011 from the water groove 1016 of the outer sheath 1011. The cooling liquid flows along the cooling flow path to one end of the outer sheath 1011 away from the gear speed changing device and flows out from the gap between the outer sheath 1011 and the burr shaft 1012 to achieve flushing and cooling of the burr head 110.

The gear speed changing device comprises a driving gear 220 and a driven gear 230, the transmission shaft 210 of the driving assembly is in transmission connection with the output end of the micro-motor 1001 through the connecting male header 1006 and the connecting female header 1004, the driving gear 220 is coaxially mounted on the transmission shaft 210, the driven gear 230 is engaged with the driving gear 220, the burr shaft 1012 is coaxially and fixedly connected to the driven gear 230, the rotating shaft of the driven gear 230 is rotatably mounted through the bearing 240, under the action of the micro-motor 1001, the transmission shaft 210 is driven to rotate, then the driving gear 220 is driven to rotate, and the driven gear 230 engaged with the driving gear 220 is also rotated, so as to drive the burr shaft 1012 to rotate. It should be noted that the variable speed rotation can also be realized by adjusting the gear ratio of the driving gear 220 and the driven gear 230, for example, when the rotating speed of the external motor is low, the gear ratio of the driving gear 220 and the driven gear 230 can be used to improve the rotating speed of the burr shaft 1012. It is capable of saving the cost of the motor and shortening the operation time, and realizing the regular and stable wound surface of the bone tissue grinding position, which is good for improving the treatment effect of bone tissue grinding or repairing.

It should be further noted that the burr shaft 1012 is an integral structure or a split structure, the burr shaft 1012 extends outside of the shell of the burr head 110 assembly, and a gap exists between the burr shaft 1012 and the shell of the burr head 110 assembly to form the water outlet of the burr head 110 assembly. That is, there is a gap between the burr shaft 1012 and the outer sheath 1011 to form a water outlet of the burr head 110 assembly. Specifically, the burr shaft 1012 is disposed in the outer sheath 1011, a cooling channel is provided with between the burr shaft 1012 and the outer sheath 1011, one end of the burr shaft 1012 close to the burr head 110 extends outside of the outer sheath 1011 and forms a water outlet.

In one possible embodiment, the integral structure of the burr shaft 1012 connects the burr head 110 to the output end of the gear speed changing device in transmission. A water injection passage is formed between the burr shaft 1012 and the outer sheath 1011.

In one possible embodiment, the burr shaft 1012 of the split structure comprises a metal wire 1013 and a burr shank 120, one end of the metal wire 1013 is in transmission connection with the gear speed changing device, one end of the burr shank 120 is connected to the burr head 110, the other end of the metal wire 1013 and the other end of the burr shank 120 are in transmission connection through the connecting assembly. The metal wire 1013 in this embodiment mainly plays a role of transmission, and the metal wire 1013 may be a flexible filament or a rigid thin sheath, and the material of the metal wire 1013 may be a steel wire or a nickel-titanium alloy wire. Any additional limitation on the material of the metal wire is not imposed in this embodiment.

Specifically, one side of the metal wire 1013 close to the gear speed changing device is sleeved with a hard connecting sheath 1014, the metal wire 1013 is fixedly connected to the hard connecting sheath 1014, one end of the hard connecting sheath 1014 coaxially passes through the rotating shaft of the driven gear 230 and is fixedly connected to the rotating shaft of the driven gear 230, so as to realize the transmission connection between the metal wire 1013 and the driven gear 230. The other end of the metal wire 1013 is fixedly connected to the burr shank 120 through the connecting assembly.

One side of the hard connecting sheath 1014 far away from the gear speed changing device enters into the outer sheath 1011, a structure supporting sheath 1015 is disposed between the hard connecting sheath 1014 and the outer sheath 1011, the structure supporting sheath 1015 is fixedly connected to the inner wall of the outer sheath 1011. The structure supporting sheath 1015 is fixedly connected to the front shell 1007, and the structure supporting sheath 1015 is located inside the front shell 1007, and part or all of the structure supporting sheath 1015 is located at one side of the water groove 1016 of the outer sheath 1011 close to the gear speed changing device, cooling water is prevented from entering the gear speed changing device. It should be noted that the structural supporting sheath 1015 in the present embodiment is preferably a plastic sheath, and the conventional metal sheath does not have a corresponding effect.

A waterproof assembly is disposed between the cooling system integrated within the burr head 110 assembly and the gear speed changing device. The waterproof assembly comprises a first waterproof sheath 1017 and a second waterproof sheath 1018, both of the first waterproof sheath 1017 and the second waterproof sheath 1018 are sleeved between the hard connecting sheath 1014 and the structural supporting sheath 1015. The first water-proof sheath 1017 and the second water-proof sheath 1018 are disposed at intervals on the hard connecting sheath 1014, and the first water-proof sheath 1017 is close to the gear speed changing device. A lining sheath 1019 is provided between the second waterproof sheath 1018 and the structure supporting sheath 1015, the lining sheath 1019 and the end surfaces of the second waterproof sheath 1018 and the structure supporting sheath 1015 form a stepped sealing structure, and the inner wall of the lining sheath 1019 covers the end part of the hard connecting sheath 1014 far away from the gear speed changing device, so as to prevent the cooling liquid from entering the gear speed changing device from the gap between the hard connecting sheath 1014 and the structure supporting sheath 1015.

Further, the outer sheath 1011 and the structure supporting sheath 1015 are provided with a dredging hole 1032, and the dredging hole 1032 on the outer sheath 1011 is connected to the dredging hole 1032 on the structure supporting sheath 1015, wherein The dredging hole 1032 on the outer sheath 1011 is connected to the outside environment, and the dredging hole 1032 on the structural supporting sheath 1015 is located between the first waterproof sheath 1017 and the second waterproof sheath 1018. In actual use of the product, when the sealing structure formed by the second waterproof sheath 1018 fails, the cooling liquid partially entering between the first waterproof sheath 1017 and the second waterproof sheath 1018 can flow out through the dredging hole 1032 on the structure supporting sheath 1015 and the dredging hole 1032 on the outer sheath 1011, and the first waterproof sheath 1017 can prevent the remaining cooling liquid from entering into the product device, realizing the fail-safe of the waterproof assembly, further improving the waterproof performance of the product.

It should be noted that the steel wire hose between the lining sheath 1019 and the burr shank 120 is sleeved with a structure holding sheath 1020, which ensures the stability of the rotation of the steel wire hose, there is a small gap between the structure holding sheath 1020 and the steel wire hose. There is also a small gap between the structure holding sheath 1020 and the lining sheath 1019, and the cooling liquid inside the outer sheath 1011 can enter the structure holding sheath 1020 to cool and lubricate the steel wire hose. A cooling liquid passage is formed between the structure holding sheath 1020 and the outer sheath 1011.

In a possible embodiment, the connecting assembly comprises a transmission connecting sheath 1027, the transmission connecting sheath 1027 is sleeved with a burr shank 120 and a metal wire 1013, the transmission connecting sheath 1027 is respectively fixedly connected to the burr shank 120 and the metal wire 1013, the metal wire 1013 is sleeved with a fail-safe sheath 1028, the transmission connecting sheath 1027 extends into the fail-safe sheath 1028, the outer wall of the transmission connecting sheath 1027 is fixedly connected to a fail-safe block 1029, the failure anti-sliding block is embedded in the inner wall of the fail-safe sheath 1028. Due to the combination of the fail-safe block and the fail-safe sheath 1028, when the transmission connection between the burr shank 120 and the metal wire 1013 fails, the burr shank 120 and the burr head 110 will not fall off, so as to ensure the safety of the operation. Further, an outer supporting sleeve 1030 is sleeved on the transmission connecting sheath 1027, the outer supporting sleeve 1030 is fixedly connected to the transmission connecting sheath 1027, and both the outer supporting sleeve 1030 and the transmission connecting sheath 1027 extend out of the outer sheath 1011. The outer supporting sleeve 1030 is sleeved with a water injection flow passage sheath 1031, the water injection flow passage sheath 1031 is located between the outer sheath 1011 and the outer supporting sleeve 1030, on one hand, the burr shank 120 is supported to ensure the stability of the movement of the burr shank 120, on the other hand, the water injection passage on the water injection flow passage sheath 1031 is connected to the cooling liquid passage inside the outer sheath 1011, so that the cooling liquid can flow out of the outer sheath 1011 to flush and cool the burr head 110. Further, when the metal wire 1013 is connected to the transmission connecting sheath 1027, the metal wire 1013 can be directly inserted into the transmission connecting sheath 1027 and connected by gluing and welding, or a transition connecting sheath can be sleeved on the metal wire 1013, The end portion of the metal wire 1013 having the transition connection sheath is extended into the transmission connection sheath 1027 and connected by gluing and welding.

In another possible embodiment, the connecting assembly comprises a transmission connecting sleeve 1021, one end of the burr shank 120 far away from the burr head 110 is provided with a deep connecting hole 1022, one end of the transmission connecting sleeve 1021 extends into the deep connecting hole 1022 and is fixedly connected to the inner wall of the deep connecting hole 1022, one end of the transmission connecting sleeve 1021 located at the outside of the deep connecting hole 1022 is a fail-safe sleeve 1023. The end surface of the fail-safe sleeve 1023 is fixedly connected to the end surface of the burr shank 120. The metal wire 1013 extends into the transmission connecting sleeve 1021 and has its end part protruding, the metal wire 1013 is fixedly connected to the inner wall of the transmission connecting sleeve 1021, so as to realize the reliable transmission connection of the metal wire 1013 and the burr shank 120. If the burr shank 120 fails, that is, the transmission connection between the metal wire 1013 and the burr shank 120 is disconnected, the burr shank 120 and the burr head 110 as a whole can be prevented from falling off by the fail-safe sleeve 1023, thereby improving the safety. Further, the outer part of the burr shank 120 is sleeved with an elastic sheet sheath 1024, the outer part of the elastic sheet sheath 1024 is sleeved with a water passing sleeve 1025, a supporting sleeve 1026 is disposed between the water passing sleeve 1025 and the shell of the burr head 110 assembly, the shell of the burr head 110 assembly is an outer sheath 1011, the elastic sheet sheath 1024 is fixedly connected to the burr shank 120, the elastic sheet sheath 1024 is rotatably connected to the water passing sleeve 1025, the supporting sleeve 1026 is fixedly connected to the outer sheath 1011. On the one hand, the burr shank 120 is supported to suppress the vibration caused by the movement of the burr shank 120, and on the other hand, the water passing passage on the water passing sleeve 1025 is connected to the cooling liquid passage inside the outer sheath 1011, so that the cooling liquid can flow out of the outer sheath 1011 to wash and cool the burr head 110.

In this embodiment, by connecting the burr head 110 assembly with the cooling system inside the power source assembly, the micro-motor 1001, the burr shaft 1012 and the burr head 110 can be cooled at the same time by injecting water once, and the cooling effect is good. The detachable burr shaft 1012 can utilize the fail-safe device to ensure that the burr shank 120 and the burr head 110 will not fall from the outer sheath 1011 after the burr shank 120 and the metal wire 1013 fail , which improves the safety of the operation. The first waterproof sheath 1017, the second waterproof sheath 1018, the structure supporting sheath 1015 and the lining sheath 1019 cooperate to isolate the gear speed changing device from the cooling system, so that the cooling liquid will not enter the gear speed changing device, and the service life of the device is improved.

### Embodiment 2

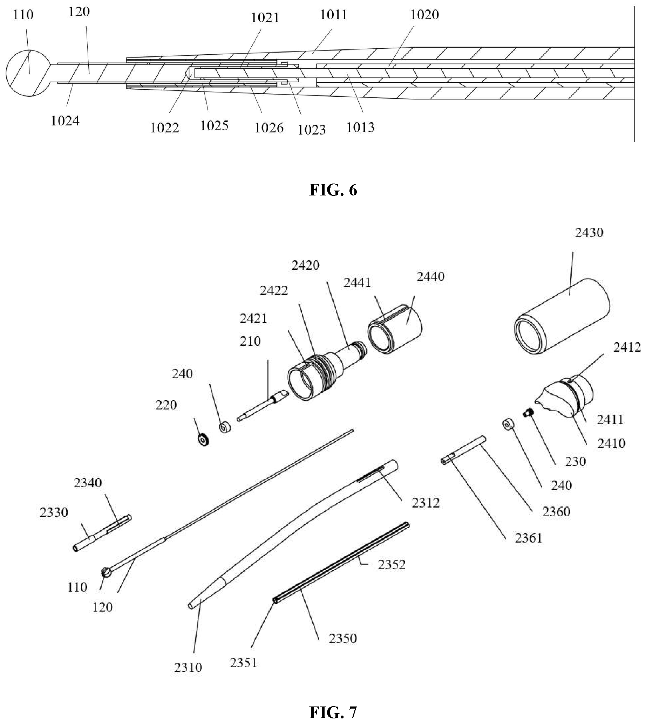

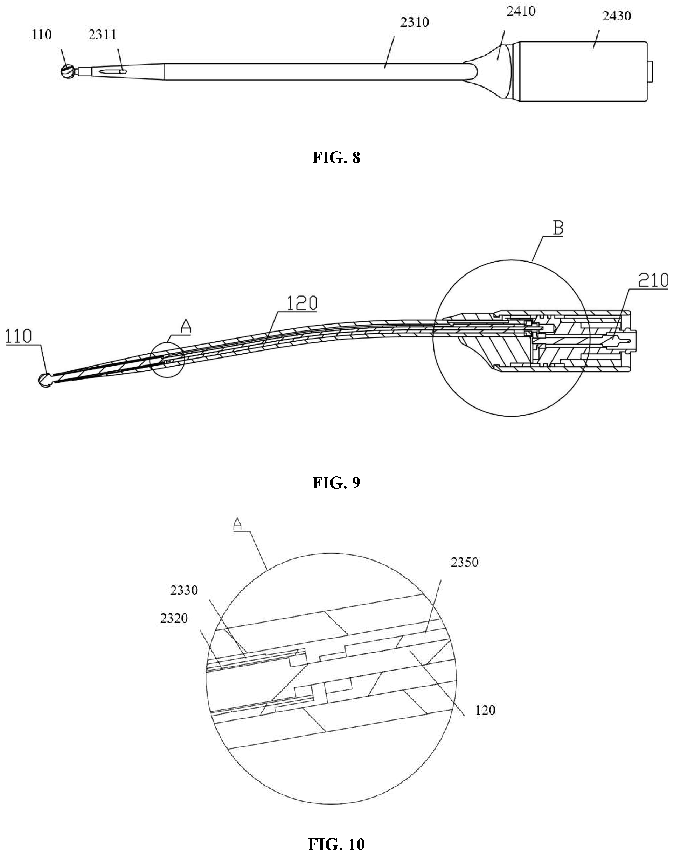

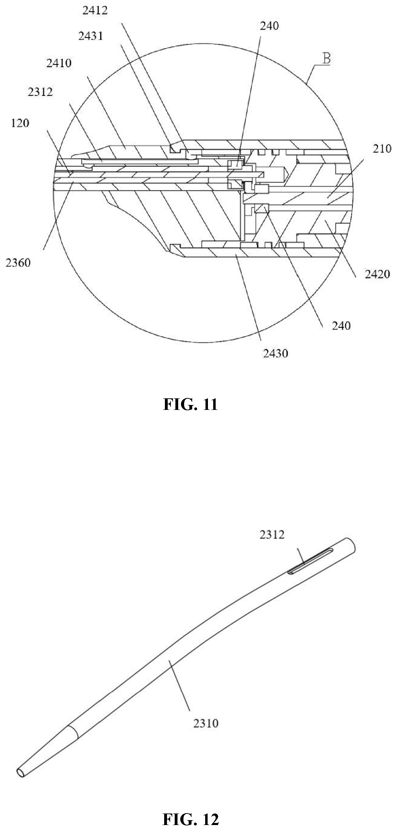

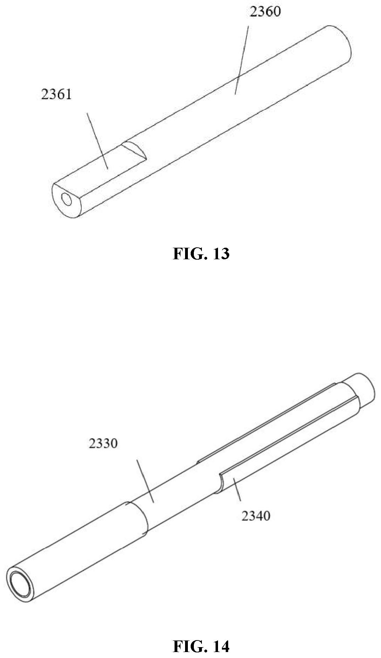

A medical bone burr is provided by the present disclosure, as shown in FIGS. 7 to 14. It is necessary to be explained in advance, the medical bone burr of the embodiment is equivalent to the unidirectional bone burr in the Embodiment 1, and comprises a bone burr assembly, a drive assembly and a supporting assembly, the bone burr assembly is equivalent to the burr assembly of first embodiment, and further comprises a front shell 2410, a spiral water-cooling interface 2420 and a sealing sleeve 2430. The bone burr assembly is used for grinding bone tissue, the bone burr assembly includes a burr head 110 and a bendable burr shank 120, the burr head 110 is mounted at one end of the burr shank 120, the driving assembly is used for driving the bone burr assembly to rotate, the driving assembly comprises a transmission shaft 210 and a transmission assembly, the power input end of the transmission assembly is connected to the transmission shaft 210, the power output end of the transmission assembly is connected to the burr shank 120. The power input end of the transmission shaft 210 is connected to an external power mechanism (such as a motor), and the transmission shaft 210 is disposed through the spiral water-cooling interface 2420. The supporting assembly comprises an outer sheath 2310 and an elastic sheet sheath 2320, the outer sheath 2310 sleeves the burr shank 120, a special tool is used for bending the outer sheath 2310 to a preset angle, so that the bone burr has a certain damping effect in the working process. The elastic sheet sheath 2320 sleeves the burr shank 120 and is close to the burr head 110. The elastic sheet sheath 2320 is provided with a damping elastic sheet, specifically, the elastic sheet sheath 2320 is provided with an opening, the opening is provided with an inclined elastic sheet. Generally, the design of the burr shank 120 is long and thin, when the burr shank 120 is rotating, a larger runout in the middle part of the burr shank 120 will definitely be caused, thereby influencing the grinding of the bone burr, however, the elastic sheet set on the elastic sheet sheath 2320 is propped against the outer wall of the burr shank 120, so that the runout space for the rotation of the burr shank 120 is limited. That is, the runout amplitude is reduced, and the smooth rotation of the burr head 110 is facilitated.

The transmission assembly comprises a driving gear 220 and a driven gear 230 engaged with each other, the driving gear 220 is mounted on the transmission shaft 210, and the driven gear 230 is mounted at one end of the burr shank 120 far away from the burr head 110. In order to enhance the stability of the burr shank 120 during rotation and to reduce friction damage, a bearing 240 is mounted between the outer sheath 2310 and the burr shank 120, and the bearing 240 is located at one end of the outer sheath 2310 close to the driven gear 230.

A water injection passage is formed between the outer sheath 2310 and the burr shank 120, one end of the outer sheath 2310 close to the burr head 110 is provided with a water outlet hole 2311, one end of the outer sheath 2310 far away from the burr head 110 is provided with a water supply groove 2312, the water outlet hole 2311 and the water supply groove 2312 are connected to the water injection channel.

A water passing sheath 2330 is further mounted between the outer sheath 2310 and the burr shank 120, the water passing sheath 2330 sleeves the elastic sheet sheath 2320, the water passing sheath 2330 comprises a big sheath and a small sheath which are fixedly connected, the big sheath is close to the burr head 110, and a part of the outer wall of the large sheath inserted into the outer sheath 2310 is adhered to the inner wall of the outer sheath 2310 to form a sealing structure, the small sheath is sleeved with a first arc-shaped sleeve 2340, the arc-shaped outer wall of the first arc-shaped sleeve 2340 is adhered to the inner wall of the outer sheath 2310. The first arc-shaped sleeve 2340 sleeves the small sheath to form the axial gap and the axial gap is connected to the water outlet hole 2311.

A supporting sleeve 2350 is also mounted between the outer sheath 2310 and the burr shank 120, the supporting sleeve 2350 comprises a sleeve 2351 and a second arc-shaped sleeve 2352 sleeved on the sleeve 2351, the sleeve 2351 sleeves the burr shank 120. The arc-shaped outer wall of the second arc-shaped sleeve 2352 is attached to the inner wall of the outer sheath 2310, and the gap formed between the second arc-shaped sleeve 2352 and the outer sheath 2310 forms a part of water injection channel, and is connected to the gap between the first arc-shaped sleeve 2340 and the outer sheath 2310. A supporting sheath 2360 is further mounted between the outer sheath 2310 and the burr shank 120, the supporting sleeve 2350 is located between the water passing sheath 2330 and the supporting sheath 2360, one end of the supporting sheath 2360 is provided with a flat square 2361, the water outlet of the water passing groove 2312 is located on the flat square 2361.

The front shell 2410 is hermetically connected to the sealing sleeve 2430, specifically, the outer wall of the front shell 2410 is provided with an annular clamping groove 2411, the sealing sleeve 2430 is provided with a clamping ring 2431, the clamping ring 2431 is embedded in the clamping groove 2411 and sealed by glue.

The outer sheath 2310 and one end of the burr shank 120 far away from the burr head 110 are both inserted into the front shell 2410, the water supply groove 2312 is located in the front shell 2410, the outer wall of the spiral water-cooling interface 2420 is provided with a spiral channel 2422 and an axial through groove 2421, the front shell 2410 is provided with a water supply hole 2412, the water supply hole 2412 is located in the sealing sleeve 2430, the water inlet end of the axial through groove 2421 is connected to the water flow channel 2422, the water outlet end of the axial through groove 2421 is connected to the water supply hole 2412. The water-cooling sleeve 2440 is further provided between the spiral water-cooling interface 2420 and the sealing sleeve 2430, the water-cooling sleeve 2440 sleeves one end of the spiral water-cooling interface 2420 far away from the front shell 2410, the water-cooling sleeve 2440 is provided with an axial water groove 2441, The axial water groove 2441 connects to the water inlet end of the water flow passage 2422. The inner wall of the front shell 2410 and the water supply groove of the outer sheath 2310, and the inner wall of the outer sheath 2310 and the flat square 2361 on the supporting sheath 2360 form a cavity through which the cooling water flows. It should be noted that the technical solution of the present disclosure does not specifically limit the shape of the water flow channel 2422, and the water flow channel 2422 may be a spiral channel, and the water flow channel 2422 may also be other regular or irregular channels, such as linear channels.

It should be noted that the design of the cooling flow passages in the outer sheath 2310 and the sealing sleeve 2430 in the Embodiment 2 is also applicable to the technical solution of the first embodiment. The water outlet hole 2311 located on the outer sheath 2310 may also be provided at the end of the outer sheath 2310 close to the burr head 110 on the basis of the cooling flow passages in the outer sheath 2310 and the sealing sleeve 2430 of the second embodiment.

In the unidirectional bone burr of the embodiment, the power input end of the transmission shaft 210 is connected to an external power mechanism (such as motor), under the action of the external power mechanism, the transmission shaft 210 is drived to rotate, so the driving gear 220 is drived to rotate. The driven gear 230 engaged with the driving gear 220 also rotates with it, so the burr shank 120 is drived to rotate. In the embodiment, the transmission assembly can further realize variable speed rotation by adjusting the gear ratio of the driving gear 220 and the driven gear 230, for instance, when the rotating speed of the outer electric motor is low, a small gear ratio of the driving gear 220 and the driven gear 230 can be used to improve the rotating speed of the burr shank 120. It is capable of saving the cost of the motor and shortening the operation time, and realizing the regular and stable wound surface of the bone tissue grinding position, which is good for improving the treatment effect of bone tissue grinding or repairing.

In the unidirectional bone burr of the embodiment, through the external water injection mechanism, cooling water is injected into the axial water groove 2441 on the water-cooling sleeve 2440, the cooling water flows into the spiral channel 2422 through the axial water groove 2441, then flows into the water supply hole 2412 of the front shell 2410 through the axial through groove 2421, then enters into the water supply groove 2312 through the water supply hole 2412, then flows from the outlet of the water supply groove 2312 to the flat square 2361 on the supporting sheath 2360, flows into the gap between the second arc-shaped sleeve and the inner wall of the outer sheath 2310 through the gap between the flat square 2361 and the outer sheath 2310, then flows through the gap between the water passing sheath 2330 and the outer sheath 2310, and finally flows out from the water outlet hole of the outer sheath 2310, thereby cooling and washing the grinding position, which is good for improving the comfort of the patient, and improving the grinding efficiency.

### Embodiment 3

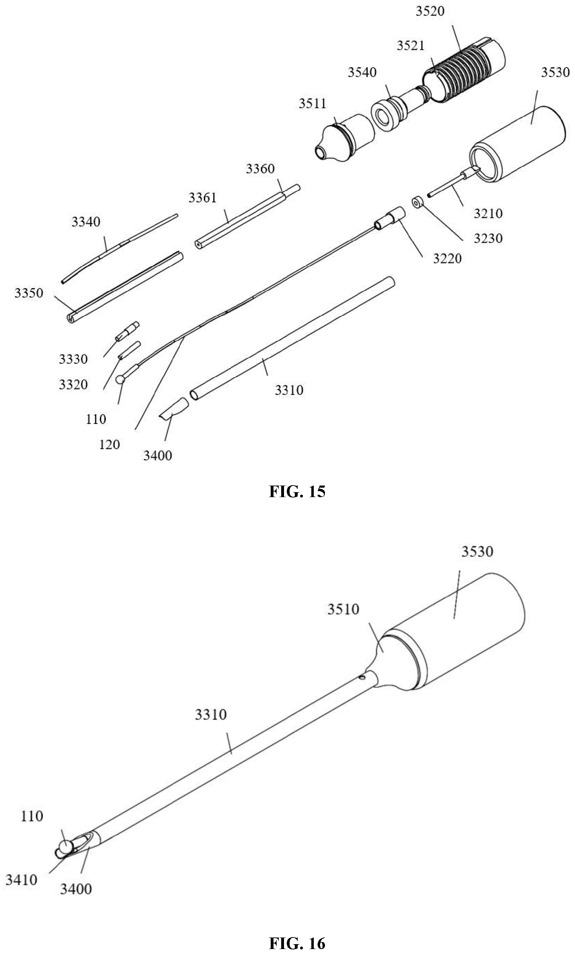

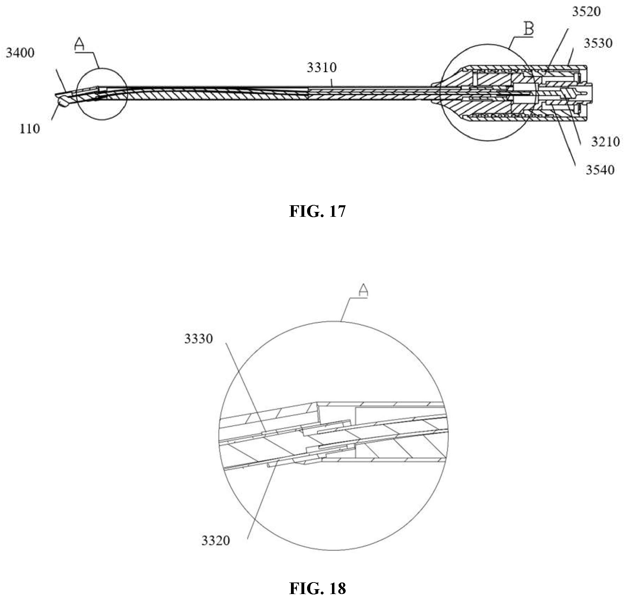

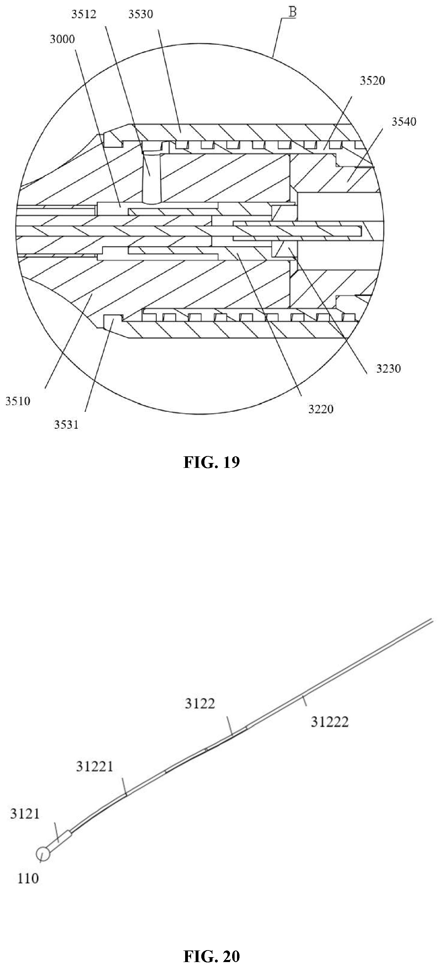

An orthopaedic bone burr is provided by the present disclosure, as shown in FIGs. 15 to 22, it should be noted that the orthopaedic bone burr in the embodiment is equivalent to the unidirectional bone burr in the Embodiment 1, and comprises a burr assembly, a driving assembly, a supporting assembly and a sheath sleeve 3400, and a front shell 3510, a water-cooling sleeve 3520 and a sealing sleeve 3530. The burr assembly is used for grinding bone tissue, the burr assembly includes a burr head 110 and a burr shank 120, the burr head 110 is mounted at one end of the burr shank 120, the burr shank 120 is elastic and bendable. The drive assembly is used for driving the burr head 110 assembly to rotate. The supporting assembly comprises an outer sheath 3310, the first sleeve 3320 and the first supporting sheath 3330, the outer sheath 3310 and the first sleeve 3320 are all sleeved on the burr shank 120, the first sleeve 3320 is close to the burr head 110, the first supporting sheath 3330 sleeves the first sleeve 3320, and the first sleeve 3320 is in clearance fit with the first supporting sheath 3330. The sheath 3400 is used to conceal and wrap a portion of the burr head 110 so as to protect the surrounding of the bone tissue when the bone tissue is grinded and to increase the safety of the operation to a certain extent. The sheath sleeve 3400 is connected to the outer sheath 3310, the axial line of the sheath sleeve 3400 is crossed with that of the outer sheath 3310, namely the sheath sleeve 3400 is connected to the outer sheath 3310 to form a bend with a certain angle.

The burr shank 120 comprises a connecting shank 3121 and a long shank 3122, the diameter of the connecting shank 3121 is greater than that of the long shank 3122, one end of the connecting shank 3121 is fixedly connected to the burr head 110, and the other end is fixedly connected to the long shank 3122. The long shank 3122 includes an elastic bending section 31221 and a straight section 31222 which are fixedly connected, the elastic bending section 31221 is formed by a plurality of short shank connections, thereby improving the strength and toughness of the burr shank. The elastic bending section 31221 formed by connecting a plurality of short shanks can realize the elastic bending function of the burr shank 120. The sheath sleeve 3400 and the outer sheath 3310 are connected to form a bend with a certain angle, the elastic bending section 31221 is accordingly curved to form a bending state, which allows for accessing some hidden bone tissue part in the grinding operation process, and avoiding obstruction of the field of view, thereby facilitating observation of the operation position.

The outer surface of the first sleeve 3320 is provided with an abrasion-resistant coating, the abrasion-resistant coating is made of abrasion-resistant materials, such as silicon dioxide and so on, the first sleeve 3320 will rotate along with the burr shank 120 when the burr shank 120 rotates, therefore, the abrasion-resistant coating can reduce the friction loss caused by the rotation of the burr shank 120. The first sleeve 3320 sleeves the connecting shank 3121 and a part of the elastic bending section 31221. The first supporting sheath 3330 is clamped at the end of the outer sheath 3310 close to one end of the burr head 110.

The sheath sleeve 3400 has an abdication groove 3410 and an eccentric hole, the abdication groove 3410 is connected to the eccentric hole, one end of the first sleeve 3320 and one end of the first supporting sheath 3330 and the burr head 110 are located in the abdication groove 3410, the other end of the first sleeve 3320 and the other end of the first supporting sheath 3330 are located in the outer sheath 3310 through the eccentric hole. The first sleeve 3320 is in clearance fit with the first supporting sheath 3330, which is beneficial for reducing the diameter of the first supporting sheath 3330, so as to form a larger avoiding space between the first sleeve 3320 and the abdication groove 3410, thereby having a better field of view when grinding the tissue.

A second sleeve 3340 is further provided between the outer sheath 3310 and the burr shank 120, the second sleeve 3340 is elastic and capable of bending, the second sleeve 3340 sleeves the burr shank 120 and capable of bending along with the bending of the elastic bending section 31221 of the burr shank 120. The arrangement of the second sleeve 3340 can enhance the overall strength and toughness of the burr shank 120. The second sleeve 3340 is sleeved with an arc-shaped guide sleeve 3350, which further forms a powerful support for the burr shank 120. The arc-shaped outer wall of the arc-shaped guide sleeve 3350 is attached to the inner wall of the outer sheath 3310. Referring to FIG. 7, the arc-shaped guide sleeve 3350 is formed by providing a through slot penetrating both ends on a circular rod-shaped structure. A second supporting sheath 3360 is further provided between the outer sheath 3310 and the burr shank 120, the second supporting sheath 3360 sleeves the burr shank 120, the second sleeve 3340 is located between the first sleeve 3320 and the second supporting sheath 3360. The second supporting sheath 3360 is used to support a section of the burr shank 120 that is remote from the burr head 110. The internal path of the arc-shaped guide sleeve 3350 first ascends and then descends from one end far away from the burr head 110 to one end close to the burr head 110 , the second sleeve 3340 conforms to the arc-shaped contour inside the arc-shaped guide sleeve 3350.

The outer wall of the second supporting sheath 3360 is provided with a flat square 3361, the gap between the flat square 3361 and the outer sheath 3310, and the gap between the arc-shaped guide sleeve 3350 and the outer sheath 3310 form a water injection channel through which the cooling water flows. It should be noted that the second supporting sheath 3360 in the embodiment is a plastic sheath, and the conventional metal sheath cannot achieve the corresponding effect.

The drive assembly includes an input shaft 3210, one end of the burr shank 120 remote from the burr head 110 is connected to the input shaft 3210, and specifically, one end of the straight section 31222 of the burr shank 120 is inserted into the input shaft 3210. The driving assembly further comprises a bearing sleeve 3220, one end of the bearing sleeve 3220 is internally mounted with a bearing 3230, the bearing sleeve 3220 is disposed on the power output end of the input shaft 3210, the disposition of the bearing 3230 is good for the burr shank 120 to rotate more stably and can reduce friction damage.

The front shell 3510 has a stepped hole inside, and the ends of the outer sheath 3310, the burr shank 120 and the second supporting sheath 3360, which are far away from the burr head 110, are inserted into the front shell 3510 from the small holes of the stepped hole. The front shell 3510 and the sealing sleeve 3530 are hermetically connected, specifically, the outer wall of the front shell 3510 is annularly provided with a clamping groove 3511, the sealing sleeve 3530 is provided with a clamping ring 3531, the clamping ring 3531 is embedded in the clamping groove 3511 and sealed by glue. The sealing sleeve 3530 sleeves the water-cooling sleeve 3520, one end of the water-cooling sleeve 3520 sleeves the front shell 3510, the outer wall of the water-cooling sleeve 3520 is provided with a spiral channel, the front shell 3510 is provided with a water supply hole 3512, the water inlet end of the water supply hole 3512 is connected to the water outlet end of the spiral channel, specifically, one end of the water-cooling sleeve 3520 close to the front shell 3510 is provided with a notch 3521, the position of the notch 3521 is corresponding to and connected to the water supply hole 3512. the water outlet end of the water supply hole 3512 is connected to the water injection channel, the outer wall of one end of the bearing sleeve 3220 is propped against the inner wall of the front shell 3510 to form a seal. Specifically, the outer diameter of one end of the bearing sleeve 3220 far away from the bearing is smaller than the outer diameter of one end of the mounting bearing 3230, that is, the outer wall of one end of the bearing sleeve 3220 far away from the bearing 3230 and the big hole wall of the stepped hole and the outer wall of the second supporting sheath 3360 form a cavity channel 3000, and the cavity 3000 is connected to the channel formed between the flat square 3361 and the outer sheath 3310, and the water supply hole 3512 is connected to the cavity 3000.

The water-cooling sleeve 3520 is internally provided with a water-cooling interface 3540, the water-cooling sleeve 3520 sleeves the water-cooling interface 3540, and the input shaft 3210 is penetrated in the water-cooling interface 3540.

In the unidirectional bone burr of the embodiment, the power input end of the input shaft 3210 is connected to an external power mechanism (such as a motor), the power output end of the input shaft 3210 is connected to the burr shank 120, when the input shaft 3210 rotates under the action of the external power mechanism, the burr shank 120 is accordingly rotated, in the rotating process, when the burr head 110 contacts the bone tissue and the grinding position is not confirmed, under the action of the elastic force, there will be an abdication flick with the elastic bending section 31221 of the burr shank 120, after the grinding position is confirmed, the burr head 110 is controlled to extrude the grinding position. The elastic bending section 31221 is bent so as to ensure the accuracy of the grinding position to a certain extent, and meanwhile, it can guide to provide a better operation angle. The disposition of the sheath sleeve 3400 can shield part of the grinding position of the burr head 110, effectively protecting the soft tissue at the non-operative site, and the disposition of the abdication groove 3410 can avoid the rotation of the burr head 110 to generate interference with the sheath sleeve 3400, so as to adapt to the configuration of the burr head 110 with a larger size. The larger the area of the burr head 110 protruding from the sheath sleeve 3400 on one side, the easier it is to adjust the angle of the grinding operation, thereby improving the working efficiency.

### Embodiment 4

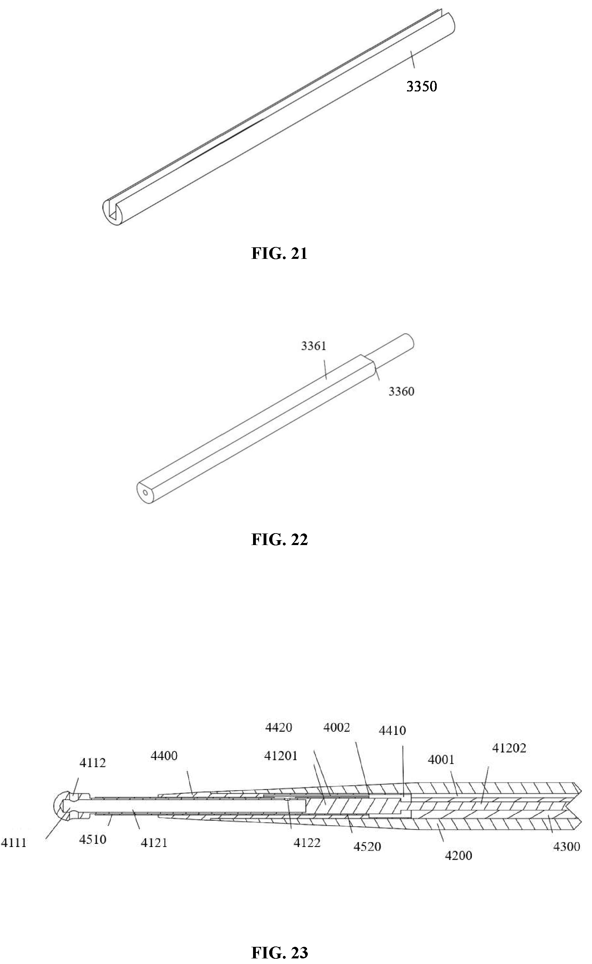

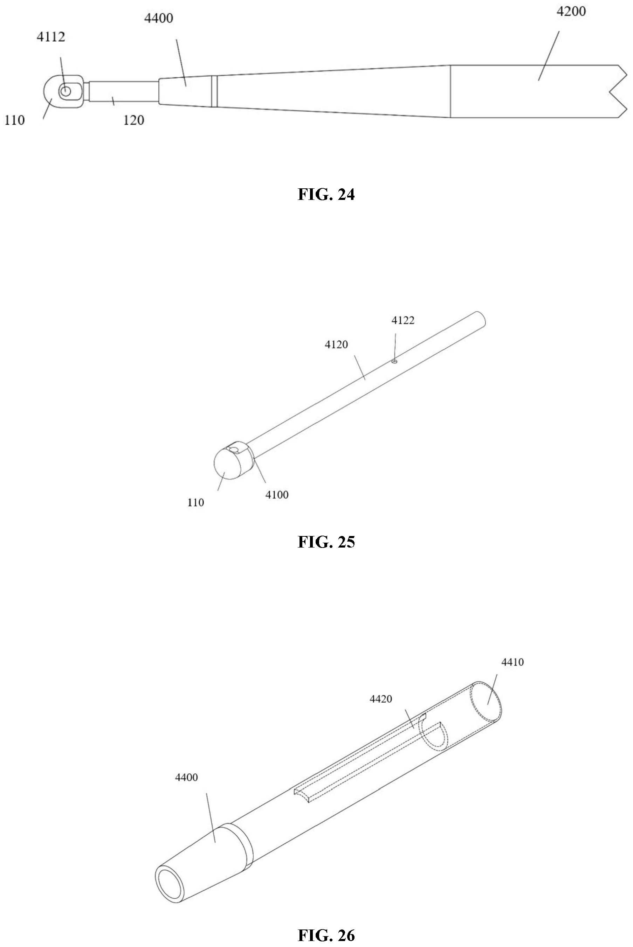

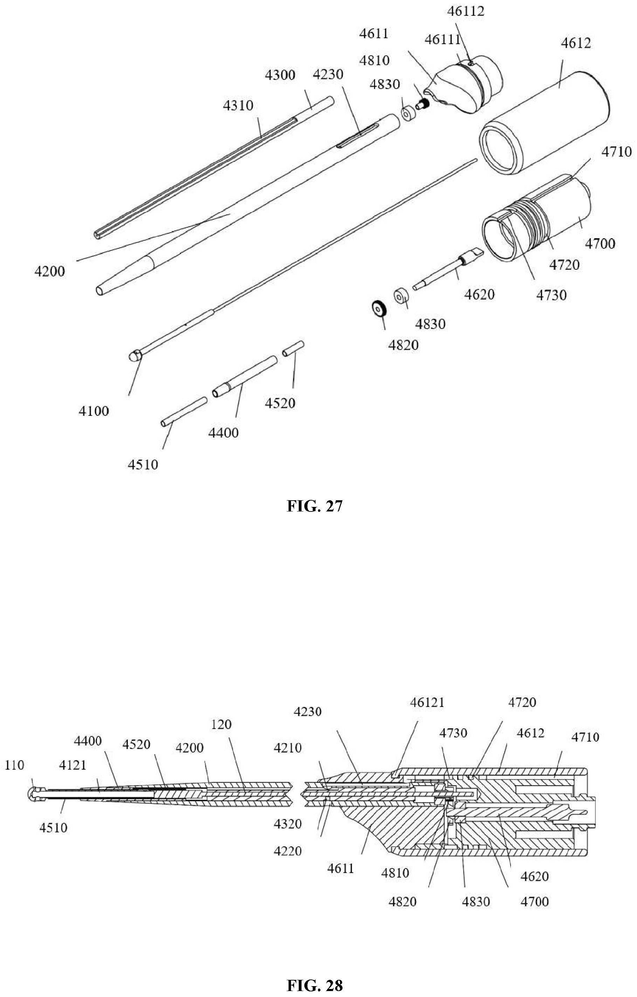

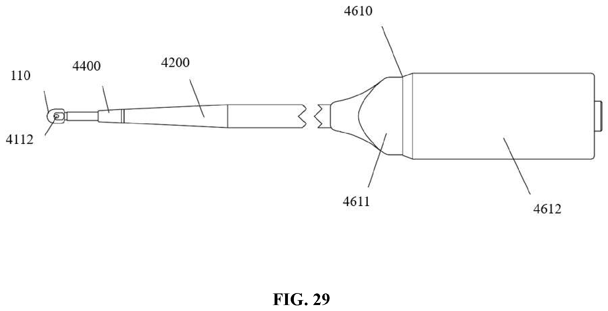

A water injection burr head mechanism and an orthopaedic bone burr are provided by the present disclosure, as shown in FIGs. 23 to 29. It should be noted that the water injection burr mechanism in the embodiment is equivalent to the unidirectional bone burr in the Embodiment 1, and comprises a burr assembly 4100 and an outer sheath 4200, the burr assembly 4100 comprises a burr head 110 and a burr shank 120 connected to the burr head 110, the burr head 110 is provided with a first cavity 4111 inside, the burr head 110 is provided with a water outlet hole 4112, the water outlet hole 4112 is connected to the first cavity 4111. One end of the burr shank 120 close to the burr head 110 has a second cavity 4121, and the second cavity 4121 connects to the first cavity 4111. The outer sheath 4200 sleeves the burr shank 120, the outer sheath 4200 is provided with a first water inlet hole 4210, the first water inlet hole 4210 is used for injecting water into the hollow inner part of the outer sheath 4200, a water flow channel is formed between the inner wall of the outer sheath 4200 and the outer wall of the burr shank 120. The water flow passage is connected to the first water inlet hole 4210 and the second cavity 4121.

In order to better arrange the second cavity 4121 inside the burr shank 120, the burr shank 120 comprises a first section 41201 and a second section 41202, the first section 41201 of the burr shank 120 is connected to the burr head 110, The outer diameter of the first section 41201 of the burr shank 120 is greater than that of the second section 41202 of the burr shank 120, and the second cavity 4121 is located in the first section 41201 of the burr shank 120.

The water injection burr head 110 mechanism further comprises a supporting guide sheath 4300, the supporting guide sheath 4300 sleeves the burr shank 120, and the inner wall of the outer sheath 4200 is tightly contacted with the outer wall of the supporting guide sheath 4300, the outer wall of the supporting guide sheath 4300 is provided with a first flat square 4310. The gap between the first flat square 4310 and the inner wall of the outer sheath 4200 forms a first channel 4001, the first channel 4001 is a part of the water flow channel, the first water inlet hole 4210 is located at one end of the first flat square 4310, and the first water inlet hole 4210 is connected to the first channel 4001.

The supporting guide sheath 4300 is further provided with a blind hole 4320, the position of the outer sheath 4200 corresponding to the blind hole 4320 is provided with a through hole 4220, the central lines of the through hole 4220 and the blind hole 4320 are overlapped, a screw or a pin is inserted into the through hole 4220 and the blind hole 4320 to realize the fixed connection between the supporting guide sheath 4300 and the outer sheath 4200, and thus the supporting guide sheath 4300 is prevented from rotating along with the burr shank 120.

A supporting cavity sheath 4400 is sleeved between the outer sheath 4200 and the burr shank 120, a second channel 4002 is provided in the supporting cavity sheath 4400, the second channel 4002 is connected to the first channel 4001, and the second channel 4002 is also a part of the water flow channel. The supporting cavity sheath 4400 and the supporting guide sheath 4300 can be separated from each other at one end or in contact with each other, and one end of the supporting guide sheath 4300 can also be inserted into the supporting cavity sheath 4400, but it must be ensured that the second channel 4002 connects to the first channel 4001.

The supporting cavity sheath 4400 is provided with a stepped hole 4410 and a guide groove 4420 therein, the stepped hole 4410 is located at one end of the supporting cavity sheath 4400 far away from the burr head 110, the guide groove 4420 is opened on the inner wall of the supporting cavity sheath 4400. The guide groove 4420 connects to the stepped hole 4410 to form the second passage 4002, and the guide groove 4420 connects to the second cavity 4121. One end of the supporting cavity sheath 4400 close to the burr head 110 extends outside of the outer sheath 4200, and the outer wall of the supporting cavity sheath 4400 extending out of the outer sheath 4200 is flush with the outer wall of the outer sheath 4200.

The burr shank 120 is provided with a second water inlet hole 4122 on the cavity wall of the second cavity 4121, and the second water inlet hole 4122 is connected to the guide groove 4420 and the second cavity 4121.

In order to make the burr shank 120 more stable in the grinding process, a front supporting sheath 4510 and a back supporting sheath 4520 are further provided between the supporting cavity sheath 4400 and the burr shank 120, the front supporting sheath 4510 sleeves one end of the burr shank 120 close to the burr head 110, one end of the front supporting sheath 4510 is close to the burr head 110, the other end is inserted in the supporting cavity sheath 4400 and avoids the second water inlet hole 4122, the back supporting sheath 4520 is also sleeved on the burr shank 120 and avoids the second water inlet hole 4122. The position of the rear supporting sheath 4520 corresponds to that of the guide groove 4420.

In the grinding process, when it is necessary to inject water and reduce temperature, water enters into the first channel 4001 from the first water inlet hole 4210 on the outer sheath 4200, flows to the second channel 4002 along the first channel 4001, flows to the guide groove 4420 from the stepped hole 4410 in the second channel 4002, enters into the second cavity 4121 through the second water inlet hole 4122, flows from the second cavity 4121 to the first cavity 4111, and flows out from the water outlet hole 4112 on the burr head 110, the water flowing out from the burr head 110 can directly act on the grinding position and the burr head 110. It is not only capable of reducing the temperature of the tissue at the grinding position, but also effectively reducing the temperature of the burr head 110, which is good for improving the cooling efficiency and cooling effect. In addition, the second cavity 4121 inside the burr shank 120 close to the burr head 110 is filled with water, which can cool the burr shank 120 during the grinding process, so as to prolong the service life of the burr head 110 assembly 100 to a certain extent.

An orthopaedic bone burr is disclosed by the embodiment, the orthopaedic bone burr of the embodiment is corresponding to the unidirectional bone burr in the Embodiment 1, and comprises the above-mentioned water injection burr head mechanism. The unidirectional bone burr also includes a shank assembly including a shell 4610 and an input shaft 4620 for connecting an external power mechanism, such as a motor, to drive the input shaft 4620 to rotate under the action of the external power mechanism. The outer sheath 4200 is inserted in the shell 4610, the input shaft 4620 is in transmission connection with the burr shank 120, the shank assembly is provided with a water inlet channel, the water outlet end of the water inlet channel is connected to the first channel 4001.

The shell 4610 comprises a first shell 4611 and a second shell 4612, the first shell 4611 is inserted in the second shell 4612, and the first shell 4611 is hermetically connected to the second shell 4612, specifically, the outer wall of the first shell 4611 is provided with an annular groove 46111. The second shell 4612 is provided with a retaining ring 46121, which is embedded in the annular groove 46111 and sealed by a sealing glue. The outer sheath 4200 is inserted in the first shell 4611, the outer wall of the outer sheath 4200 is provided with a water inlet groove 4230, the water inlet groove 4230 is connected to the first water inlet hole 4210, the water inlet groove 4230 is located in the first shell 4611. The second shell 4612 is internally provided with a water-cooling interface 4700, the water-cooling interface 4700 sleeves the input shaft 4620, the outer wall of the water-cooling interface 4700 is sequentially provided with a first water injection groove 4710, a spiral passage 4720 and a second water injection groove 4730, the first shell 610 is provided with a third water inlet hole 46112, the third water inlet hole 46112 is located in the second shell 610, the water outlet end of the second water injection groove 4730 is connected to the third water inlet hole 46112, the water outlet end of the third water inlet hole 46112 is connected to the water inlet groove 4230.

One end of the burr shank 120 far away from the burr head 110 is fixed with a driven gear 4810, the input shaft 4620 is fixed with a driving gear 4820, the driven gear 4810 is engaged with the driving gear 4820. The bearing 4830 is mounted between the input shaft 4620 and the water-cooling interface 4700, and the bearing 4830 is also mounted between the outer sheath 4200 and the burr shank 120 at a position close to the driven gear 4810. When the input shaft 4620 rotates, the driving gear 4820 accordingly rotates, and the driven gear 4810 engaged with the driving gear 4820 also rotates to drive the burr shank 120 and the burr head 110 to rotate to grind the bone tissue.

Water is injected into the first water injection groove 4710 on the water-cooling interface 4700 through the external water injection mechanism, the injected water flows through the first water injection groove 4710, a spiral channel 4720 and a second water injection groove 4730, and enters into the water inlet groove 4230 from the third water inlet hole 46112, then enters into the water flow channel through the first water inlet hole 4210 so as to flow to the second cavity 4121 and the first cavity 4111, and flows out from the water outlet hole 4112 on the burr head 110.

### Embodiment 5

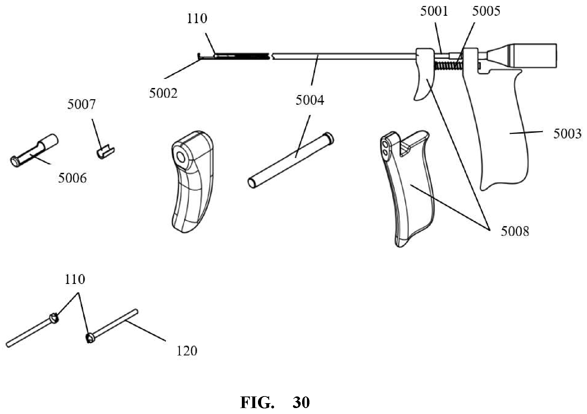

Based on Embodiment 1 or 2, as shown in FIG. 30, the disclosure further provides a rongeur, comprising a burr head 110 assembly, the burr head 110 assembly comprises a burr head 110, burr shank 120 and outer sheath 5001, burr head 110 is mounted at one end of the burr shank 120, outer sheath 5001 sleeves the burr shank 120, burr shank 120 is rotatable relative to outer sheath 5001 and drives the burr head 110 to rotate. The burr head 110 assembly of the present disclosure may employ the burr head 110 assembly of Embodiment 1.

A sheath sleeve 5002, an adjusting mechanism and a shank 5003 are further comprised, the adjusting mechanism and the burr head 110 assembly are configured on the shank 5003, the adjusting mechanism is provided with a pressing shank 5008, the adjusting mechanism controls the sheath sleeve 5002 to move back and forth relative to the burr head 110. The pressing shank 5008 is fixedly connected to a guide rod 5004, the guide rod 5004 sleeves the outer sheath 5001, the guide rod 5004 connects the sheath sleeve 5002 with the pressing shank 5008, a reset spring 5005 is provided between the pressing shank 5008 and the shank 5003. The shank 5003, the guide rod 5004 and the sheath sleeve 5002 are integrally formed to be close to or far away from the shank 5003 along the extending direction of the outer sheath 5001.

The operator manually presses the pressing shank 5008 to move the pressing shank 5008 toward the shank 5003, at which time, the sheath sleeve 5002 moves in a direction close to the burr head 110. When the pressing shank 5008 is released, the pressing shank 5008 is reset under the action of the reset spring 5005, and the sheath sleeve 5002 is moved in a direction away from the burr head 110.

Specifically, the burr head 110 is an end cutting edge structure, the sheath sleeve 5002 is a semi-surrounding structure, the inner wall shape of the end part of the sheath sleeve 5002 is matched with the end surface shape of the burr head 110, and the edge of the end part of the sheath sleeve 5002 is provided with a tip. The circumferential side wall of the sheath sleeve 5002 is provided with a guide groove 5006, and the side edge of the burr head 110 enters the guide groove 5006. A gasket 5007 is provided between the sheath sleeve 5002 and the outer sheath 5001, so that the central axis of the burr head 110 is not overlapped with that of the sheath sleeve 5002.

It should be noted that: the conventional rongeur bone burr is made of emery, the whole cutting head is capable of cutting, the technical solution of the embodiment only disposes a cutting blade at the front end of the burr head 110, the circumferential surface of the burr head 110 is smooth, in this way, the space can be further reduced, the thickness of the sheath wall of the guide groove 5006 is increased, and the strength is increased.

The reason why the side surface of the burr head 110 does not open the blade is that the burr head 110 is prevented from contacting with the guide groove 5006 to damage the guide groove 5006, and in the case that the side surface of the burr head 110 does not open the blade, the guide groove 5006 can be closer to the burr head 110, the wall of the guide groove 5006 can be designed thicker to increase the structural strength of the guide groove 5006. The hole channel in the sheath sleeve 5002 is an eccentric hole channel, the gasket 5007 can make the central axis of the burr head 110 not overlapped with that of the sheath sleeve 5002. On one hand, it can protect the side wall of the guide groove 5006; on the other hand, it can design the groove wall of the guide groove 5006 thicker, such that the structural strength of the guide groove 5006 is increased.

In the description of the present disclosure, it is to be understood that the orientation or positional relationship indicated by the terms "upper", "lower", "front", "back", "left", "right", "vertical", "horizontal", "top", "bottom", "inner", "outer" or the like is based on the orientation or positional relationship shown in the accompanying drawings, only for the purpose of facilitating the description of the present disclosure and the simplified description, rather than indicating or implying that the device or element referred to must have a specific orientation, or be constructed and operated in a specific orientation, therefore it is not to be construed as limiting the present disclosure.

The specific embodiment of the present disclosure is described above. It should be understood that the present disclosure is not limited to the specific embodiments described above, and those skilled in the art could make various changes or modifications within the scope of the appended Claims without departing from the spirit of the present disclosure. In the absence of conflict, the embodiments of the present disclosure and the features in the embodiments may be combined with each other arbitrarily.

## Claims

1. A unidirectional bone burr, **characterized by** comprising a burr assembly, the burr assembly comprises a burr head, a burr shank and an outer sheath, the burr head is mounted at one end of the burr shank, the outer sheath sleeves the burr shank; and
the burr shank is rotatable relative to the outer sheath to drive rotation of the burr head.

2. The unidirectional bone burr according to claim 1, **characterized by** further comprising a power source assembly and a cooling system, the burr assembly is detachably connected to the power source assembly;
an output end of the power source assembly outputs torque;
the burr assembly comprises the burr head and a driving assembly, the driving assembly connects the burr head and the output end of the power source assembly in transmission; and
the cooling system cools the power source assembly and the burr assembly.

3. The unidirectional bone burr according to claim 2, **characterized in that**, the cooling system is integrated inside the burr assembly and the power source assembly, a water inlet of the cooling system is disposed at one end of the power source assembly far away from the burr assembly, a water outlet of the power source assembly is connected to a water inlet of the burr assembly, a water outlet of the burr assembly is located close to the burr head to cool the burr head.

4. The unidirectional bone burr according to claim 2, **characterized in that**, the power source assembly comprises a micro-motor, a motor shell and a cooling sheath, the cooling sheath sleeves an outer part of the micro-motor, the motor shell sleeves an outer part of the cooling sheath, a cooling channel allowing the cooling liquid to pass through is formed between the cooling sheath and the motor shell, a water inlet of the cooling channel is disposed at one end of the motor shell far away from the burr assembly, a water outlet of the cooling channel is disposed at one end of the motor shell close to the burr assembly; and
a shell of the burr assembly is detachably connected to the motor shell, an output end of the micro-motor is provided with a connecting female header, a transmission shaft of the driving assembly is provided with a connecting male header, the connecting female header and the connecting male header are detachably connected.

5. The unidirectional bone burr according to claim 2, **characterized in that**, the driving assembly is disposed inside the shell of the burr assembly, the driving assembly comprises a transmission shaft and a gear speed changing device, a power input end of the gear speed changing device is connected to the transmission shaft in transmission, a power output end of the gear speed changing device is in transmission connection with the burr head through a burr shaft.

6. The unidirectional bone burr according to claim 5, **characterized in that**, the burr shaft is an integral structure or a split structure, the burr shaft extends outside of the shell of the burr assembly, and there is a gap between the burr shaft and the shell of the burr assembly to form the water outlet of the burr assembly;
the burr shaft of the integral structure connects the burr head to an output end of the gear speed changing device in transmission; and
the burr shaft of the split structure comprises a metal wire and the burr shank, one end of the metal wire is in transmission connection with the gear speed changing device, one end of the burr shank is connected to the burr head, and the other end of the metal wire is in transmission connection with the other end of the burr shank through a connecting assembly.

7. The unidirectional bone burr according to claim 6, **characterized in that**, the connecting assembly comprises a transmission connecting sleeve, one end of the burr shank far away from the burr head is provided with a deep connecting hole, one end of the transmission connecting sleeve extends into the deep connecting hole and is fixedly connected to an inner wall of the deep connecting hole, one end of the transmission connection sleeve located outside the deep connection hole is a fail-safe sleeve, an end surface of the fail-safe sleeve is fixedly connected to that of the burr head; and
the metal wire extends into the transmission connecting sleeve and has its end part protruding, the metal wire is fixedly connected to the inner wall of the transmission connecting sleeve.

8. The unidirectional bone burr according to claim 7, **characterized in that**, an outer part of the burr shank is sleeved with an elastic sheet sheath, an outer part of the elastic sheet sheath is sleeved with a water passing sleeve, a supporting sleeve is disposed between the water passing sleeve and the shell of the burr assembly.

9. The unidirectional bone burr according to claim 6, **characterized in that**, the connecting assembly comprises a transmission connecting sheath, the transmission connecting sheath is sleeved with a burr shank and a metal wire, the transmission connecting sheath is respectively fixedly connected to both the burr shank and the metal wire, the metal wire is sleeved with a fail-safe sheath, the transmission connecting sheath extends into the fail-safe sheath, an outer wall of the transmission connecting sheath is fixedly connected to a fail-safe block, the fail-safe block is embedded in an inner wall of the fail-safe sheath.

10. The unidirectional bone burr according to claim 6, **characterized in that**, a waterproof assembly is disposed between the cooling system integrated inside the burr assembly and the gear speed changing device.

11. A medical bone burr, **characterized in that**, comprising:
a bone burr assembly for grinding bone tissue, the bone burr assembly comprises a burr head and a bendable burr shank, the burr head is mounted at one end of the burr shank; and
a supporting assembly, comprising an outer sheath with a water outlet hole on a side face, the outer sheath sleeves the burr shank, a gap between the outer sheath and the burr shank forms a water injection channel, the water outlet hole is connected to the water injection channel.

12. The medical bone burr according to claim 11, **characterized by** further comprising a driving assembly, the driving assembly comprises a transmission shaft and a transmission part, a power input end of the transmission part is connected to the transmission shaft, a power output end of the transmission part is connected to the burr shank; and
the transmission part comprises a driving gear and a driven gear engaged with each other, the driving gear is mounted on the transmission shaft, the driven gear is mounted at one end of the burr shank far away from the burr head, a bearing is mounted between the outer sheath and the burr shank, the bearing is located at one end of the outer sheath close to the driven gear.

13. The medical bone burr according to claim 11, **characterized in that**, the supporting assembly further comprises an elastic sheet sheath, the elastic sheet sheath sleeves the burr shank and is close to the burr head, the elastic sheet sheath is provided with a damping elastic sheet;
the outer sheath is bent, one end of the outer sheath far away from the burr head is provided with a water supply groove, the water supply groove is connected to the water injection channel;
a water passing sheath is further mounted between the outer sheath and the burr shank, the water passing sheath sleeves the elastic sheet sheath, the water passing sheath comprises a big sheath and a small sheath which are fixedly connected, the big sheath is close to the burr head, and a part of an outer wall of the big sheath inserted into the outer sheath is adhered with the inner wall of the outer sheath, the small sheath is sleeved with a first arc-shaped sleeve, an arc-shaped outer wall of the first arc-shaped sleeve is adhered with the inner wall of the outer sheath, an axial gap formed by the first arc-shaped sleeve sleeved on the small sheath is connected to the water outlet hole;
a supporting sleeve is further mounted between the outer sheath and the burr shank, the supporting sleeve comprises a sleeve and a second arc-shaped sleeve sleeved on the sleeve, the sleeve sleeves the burr shank, an arc-shaped outer wall of the second arc-shaped sleeve is adhered to the inner wall of the outer sheath, a gap formed between the second arc-shaped sleeve and the outer sheath forms a part of the water injection channel; and
a supporting sheath is further mounted between the outer sheath and the burr shank, the supporting sleeve is located between the water passing sheath and the supporting sheath, one end of the supporting sheath is provided with a flat square, the water outlet of the water passing groove is located on the flat square.

14. The medical bone burr according to claim 11, **characterized by** further comprising a front shell, a spiral water-cooling interface and a sealing sleeve, the front shell and the sealing sleeve are hermetically connected, one end of the outer sheath and one end of the burr shank far away from the burr head are inserted into the front shell, the water supply groove is located in the front shell, an outer wall of the spiral water-cooling interface is provided with a water flow channel and an axial through groove, the front shell is provided with a water supply hole, the water supply hole is located in the sealing sleeve, the water supply hole is connected to the water supply groove, a water inlet end of the axial through groove is connected to the water flow channel, a water outlet end of the axial through groove is connected to the water supply hole; and
a water-cooling sleeve is further disposed between the spiral water-cooling interface and the sealing sleeve, the water-cooling sleeve sleeves one end of the spiral water-cooling interface far away from the front shell, the water-cooling sleeve is provided with an axial water groove, the axial water groove is connected to the water inlet end of the spiral channel.

15. An orthopaedic bone burr, **characterized by** comprising:
a burr assembly for grinding bone tissue, the burr assembly comprises a burr head and a burr shank, the burr head is mounted at one end of the burr shank, the burr shank has elasticity and can be bent;
a driving assembly for driving the burr assembly to rotate;
a supporting assembly, comprising an outer sheath and an arc-shaped guide sleeve, the outer sheath sleeves the burr shank, the arc-shaped guide sleeve is located between the outer sheath and the burr shank; and
a sheath sleeve for hiding and wrapping a part of the burr head, the sheath sleeve is connected to the outer sheath, an axial line of the sheath sleeve is crossed with that the outer sheath.

16. The orthopaedic bone burr according to claim 15, **characterized in that**, the supporting assembly further comprises a first sleeve and a first supporting sheath, the first sleeve sleeves the burr shank, and the first sleeve is close to the burr head, the first supporting sheath sleeves the first sleeve, and the first sleeve is in clearance fit with the first supporting sheath;
the sheath sleeve is provided with an abdication groove and an eccentric hole, the abdication groove is connected to the eccentric hole, the burr head and the first sleeve and one end of the first supporting sheath are located in the abdication groove;
the burr shank comprises a connection shank and a long shank, one end of the connection shank is connected to the burr head, the other end is connected to the long shank, the long shank comprises an elastic bending section and a straight section, the elastic bending section is composed of multiple short shanks;
a second sleeve is further disposed between the outer sheath and the burr shank, the second sleeve can be bent, and the second sleeve sleeves the burr shank;
the arc-shaped guide sleeve sleeves the second sleeve, an arc-shaped outer wall of the arc-shaped guide sleeve is adhered to an inner wall of the outer sheath; and
an internal path of the arc-shaped guide sleeve first ascends and then descends from one end far away from the burr head to one end close to the burr head; the second sleeve conforms to an arc-shaped contour inside the arc-shaped guide sleeve.

17. The orthopaedic bone burr according to claim 16, **characterized in that**, a second supporting sheath is further disposed between the outer sheath and the burr shank, the second supporting sheath sleeves the burr shank, the second sleeve is located between the first sleeve and the second supporting sheath; and
an outer wall of the second supporting sheath is provided with a flat square, a gap between the flat square and the outer sheath, and a gap between the arc-shaped guide sleeve and the outer sheath form a water injection passage through which the cooling water flows.

18. The orthopaedic bone burr according to claim 17, **characterized in that**, the driving assembly comprises an input shaft, one end of the burr shank far away from the burr head is connected to the input shaft; the driving assembly further comprises a bearing sleeve, one end of the bearing sleeve is mounted with a bearing, the bearing sleeve is disposed on a power output end of the input shaft; and
a front shell, a water-cooling sleeve and a sealing sleeve are further comprised, a stepped hole is disposed inside the front shell, the outer sheath, the burr shank and one end of the second supporting sheath far away from the burr head is inserted into the front shell from a small hole of the stepped hole, the front shell is hermetically connected to the sealing sleeve, the sealing sleeve sleeves the water-cooling sleeve, one end of the water-cooling sleeve sleeves the front shell, an outer wall of the water-cooling sleeve is provided with a spiral channel, the front shell is provided with a water supply hole, a water inlet end of the water supply hole is connected to a water outlet end of the spiral channel, a water outlet end of the water supply hole is connected to the water injection channel, an outer wall of one end of the bearing sleeve is propped against an inner wall of the front shell to form a seal.

19. A water injection burr head mechanism, **characterized by** comprising a burr assembly and an outer sheath, the burr assembly comprises a burr head and a burr shank connected to the burr head, a first cavity is provided inside the burr head, the burr head is provided with a water outlet hole, the water outlet hole is connected to the first cavity, one end of the burr shank close to the burr head is provided with a second cavity, the second cavity is connected to the first cavity; and
the outer sheath sleeves the burr shank, the outer sheath is provided with a first water inlet hole, the first water inlet hole is used for injecting water into the outer sheath, a water flow channel is formed between an inner wall of the outer sheath and an outer wall of the burr shank, the water flow channel is connected to both the first water inlet hole and the second cavity.

20. The water injection burr head mechanism according to claim 19, **characterized by** further comprising a supporting guide sheath, the supporting guide sheath sleeves the burr shank, and the inner wall of the outer sheath is contacted with an outer wall of the supporting guide sheath, the outer wall of the supporting guide sheath is provided with a first flat square, a gap between the first flat square and the inner wall of the outer sheath forms a first channel, the first channel is a part of the water flow channel, the first water inlet hole is located at one end of the first flat square, and the first water inlet hole is connected to the first channel;
a supporting cavity sheath is sleeved between the outer sheath and the burr shank, a second channel is disposed inside the supporting cavity sheath, the second channel is connected to the first channel, the second channel is also a part of the water flow channel;
a stepped hole and a guide groove are provided inside the supporting cavity sheath, the stepped hole is located at one end of the supporting cavity sheath far away from the burr head, the guide groove is opened on an inner wall of the supporting cavity sheath, the guide groove is connected to the stepped hole to form a second channel, the guide groove is connected to the second cavity;
the burr shank is provided with a second water inlet hole on a wall of the second cavity, the second water inlet hole is connected to both the guide groove and the second cavity; and
a front supporting sheath and a back supporting sheath are further disposed between the supporting cavity sheath and the burr shank, the front supporting sheath sleeves one end of the burr shank close to the burr head, one end of the front supporting sheath is close to the burr head, the other end is inserted into the supporting cavity sheath and keeps away from the second water inlet hole, the back supporting sheath also sleeves the burr shank and keeps away from the second water inlet hole; a position of the back supporting sheath is corresponding to that of the guide groove.

21. A rongeur, **characterized by** comprising a burr assembly, the burr assembly comprises a burr head, a burr shank and an outer sheath, the burr head is mounted at one end of the burr shank, and the outer sheath sleeves the burr shank, the burr shank is rotatable relative to the outer sheath to drive rotation of the burr head; and
further comprising a sheath sleeve, an adjusting mechanism and a shank, the adjusting mechanism and the burr assembly are configured on the shank, the adjusting mechanism is provided with a pressing shank, the adjusting mechanism controls the sheath sleeve to move back and forth relative to the burr head.

22. The rongeur according to claim 21, **characterized in that**, the pressing shank is fixedly connected to a guide rod; the guide rod sleeves the outer sheath; the guide rod connects the sheath sleeve to the pressing shank; a reset spring is disposed between the pressing shank and the shank; and
the shank, the guide rod and the sheath sleeve as a whole can move toward or away from the shank along an extending direction of the outer sheath.

23. The rongeur according to claim 21, **characterized in that**, a shape of the inner wall of an end part of the sheath sleeve is matched with that of the end surface of the burr head, and an edge of the end part of the sheath sleeve is provided with a tip, a peripheral side wall of the sheath sleeve is provided with a guide groove, a side edge of the burr head enters into the guide groove; and
a gasket is disposed between the sheath sleeve and the outer sheath such that a central axial line of the burr head is not overlapped with that of the sheath sleeve.

24. The rongeur according to claim 21, **characterized in that**, a front end of the bone burr is provided with a cutting edge, while its circumferential surface is smooth.
